# EUROPEAN PATENT SPECIFICATION

(11) **EP 4 327 840 B1**
(45) Date of publication and mention of the grant of the patent: **04.03.2026**
(21) Application number: 23210952.0
(22) Date of filing: 30.10.2019
(51) Int. Cl.: A61L 26/00, A61L 31/04, A61L 31/14, A61L 24/00, A61K 31/717, A61K 45/06, A61L 24/08, A61P 41/00

(54) **COMPOSITIONS COMPRISING OXIDIZED CELLULOSE**
ZUSAMMENSETZUNGEN MIT OXIDIERTER CELLULOSE
COMPOSITIONS COMPRENANT DE LA CELLULOSE OXYDÉE

(30) Priority: 01.11.2018 IL 26271618; 01.11.2018 US 201862753981 P
(43) Date of publication of application: 28.02.2024
(62) Divisional of application: 19806061.8
(73) Proprietor: Omrix Biopharmaceuticals Ltd., Rehovot 7610601 (IL)
(72) Inventor: ILAN, Erez, 7039500 Kibbutz Netzer Sereni (IL); FAINGOLD, Omri, 7634940 Rehovot (IL); MONTIA, Eve, 7632848 Rehovot (IL); ALPERIN, Hadas, 6731935 Tel-Aviv (IL)
(74) Representative: Carpmaels & Ransford LLP

(56) References cited:
- EP-A1- 1 559 438
- WO-A1-2004/080500
- WO-A1-2007/106251
- WO-A1-2013/177242
- US-A- 4 668 224
- US-A- 5 134 229
- US-A1- 2003 073 663
- US-B2- 9 539 358

## Description

### FIELD OF THE INVENTION

The invention relates to compositions comprising oxidized cellulose (OC) for use as an adhesion prevention material.

### BACKGROUND OF THE INVENTION

In various situations during surgery therapeutic agents are spread inside the human body, for prevention of bleeding or adhesions after surgery. Existing agents have drawbacks, for example, sheets are difficult to place in minimal invasive surgeries.

The selection of appropriate methods or products for the control of bleeding and/or adhesions is dependent upon many factors, which include but are not limited to bleeding severity, anatomical location of the source and the proximity of adjacent critical structures, whether the bleeding is from a discrete source or from a broader surface area, visibility and precise identification of the source and access to the source.

In an effort to address the above-described problems, materials have been developed for both adhesions prevention and controlling excessive bleeding. Topical Absorbable Hemostats (TAHs) are widely used in surgical applications. TAHs encompass products based on oxidized cellulose (OC), gelatin, collagen, chitin, chitosan, etc. To improve the hemostatic performance, scaffolds based on the above materials can be combined with biologicallyderived clotting factors, such as thrombin and fibrinogen. To prevent adhesions formation, several products are commercially available. Some of the adhesion barriers are based on oxidized cellulose (OC), modified sugars and modified starch.

Due to its biodegradability, and its bactericidal and hemostatic properties, oxidized cellulose (OC) based materials such as oxidized regenerated cellulose (ORC), have long been used as topical hemostats. OC and ORC based materials are also used as an adhesion barrier. Products based on ORC are used in a variety of surgical procedures including: neurosurgery, abdominal surgery, cardiovascular surgery, thoracic surgery, head and neck surgery, pelvic surgery and skin and subcutaneous tissue procedures. Several methods for forming various types of hemostats based on oxidized cellulose materials are known, whether made in powder, woven, non-woven, knit, and other forms. Currently utilized hemostats include powder, or fabrics comprising ORC.

However, since adhesions prevention and control of bleeding are essential and critical in surgical procedures to minimize blood loss, to reduce post-surgical complications, and to shorten the duration of the surgery in the operating room, there is a need for improved forms and materials which facilitate ease of application, especially in hard-to-reach areas.

US9447196B2 discloses a process for dissolving modified cellulose including contacting a modified cellulose solution with at least one non-solvent to form a plurality of modified cellulose particles.

US9572907 describes implantable medical devices containing polymeric film layer consisting of glycerol and carboxymethylcellulose.

EP3258974 describes a hemostatic composition comprising water-retaining, binder dust suppression, and inorganic and organic hemostatic agents.

US6627749 discloses a controlled chemical method to produce oxidized cellulose in high yields (75-95%) and different levels of oxidation (carboxyl content <25.6%, w/w), suitable for use as an immobilizing matrix or carrier for drugs, chemicals, and biological macromolecules

US20060008505 discloses a delivery system for a hemostatic material comprising a self-adhesive strip of a bio-adhesive, especially pectin, and a glycerol plasticizer.

WO2013049049 discloses adhesion prevention fabrics prepared from oxidized regenerated cellulose.

INTERCEED (Johnson & Johnson Patient Care Inc., New Brunswick, NJ) is an absorbable fabric specially designed to reduce postsurgical adhesions. (FERTILITY AND STERILITY Vol. 51, No.6, June 1989 INTERCEED(TC7) Adhesion Barrier Study Group).

US 2003/073663 A1 describes bioabsorbable medical devices prepared by oxidizing derivatives of cellulose. Paragraph [0007] states that "a cloth of oxidized regenerated cellulose (INTERCEED available from Johnson & Johnson Medical, Inc.) has been approved by the United States Food and Drug Administration for reducing surgical adhesions in certain pelvic procedures and has been shown to have a general adhesion limiting effect in other surgical procedures".

### SUMMARY OF THE INVENTION

The invention is defined by the appended claims.

The present invention relates to a composition comprising oxidized cellulose (OC) for use as an adhesion prevention material by topical application of the composition at a surgical site, wherein the OC is in the form of a powder and has a carboxyl content ranging from about 9% to about 18% by weight, and wherein the composition is suitable for preparing a spreadable paste or powder.

In one aspect, the composition comprises oxidized cellulose (OC) and glycerol, wherein
(i) the ratio of glycerol to OC is at least about 0.5:1 w/w glycerol:OC;
(ii) the total water content is less than about 8% w/w; and
(iii) the composition is in the form of a paste at one or more temperature values selected from the group consisting of 10°C, 15°C, 20°C, 25°C, 30°C, 35°C, 37°C, and 40°C.

In some embodiments, the composition has a viscosity of at least about 10% higher than that of the glycerol at one or more temperature values selected from the group consisting of 10°C, 15°C, 20°C, 25°C, 30°C, 35°C, 37°C, and 40°C.

In some embodiments, the composition has a resistance to penetration lower than about 20 N as measured in a tensile machine adjusted to monitor a probe having a 1.27 cm diameter at a speed of 30 mm/min from a defined preload of 0.1 N at 8 mm penetration at about room temperature. In some embodiments, the composition has a resistance to penetration higher than about 1 N as measured in a tensile machine adjusted to monitor a probe having a 1.27 cm diameter at a speed of 30 mm/min from a defined preload of 0.1 N at 8 mm penetration at about room temperature.

In some embodiments, the ratio of glycerol to OC is between about 0.5:1 and about 6:1 w/w glycerol:OC.

In some embodiments, the OC comprises oxidized regenerated cellulose (ORC). In some embodiments, the composition further comprises at least one biologically active agent. In some embodiments, the at least one biologically active agent is calcium. In some embodiments, the composition further comprises one or more excipients selected from the group consisting of sodium chloride, mannitol, albumin, and sodium acetate.

In some embodiments, the ratio of glycerol to OC powder is between about 0.5:1 and about 6:1 w/w glycerol:OC.

In some embodiments, the OC powder comprises oxidized regenerated cellulose (ORC) powder. In some embodiments, the composition further comprises at least one biologically active agent. In some embodiments, the at least one biologically active agent is calcium. In some embodiments, the composition further comprises one or more excipients selected from the group consisting of sodium chloride, mannitol, albumin, and sodium acetate.

Typically, powder is matter in a finely divided state, such as particulate matter. Powder can be a loose grouping or aggregation of solid particles, usually smaller than 1000 micrometers.

Absorbable oxidized regenerated cellulose nonwoven fabrics that can be used to prepare the powder include absorbable hemostats, including but not limited to SURGICEL FIBRILLAR absorbable hemostat and SURGICEL SNOW absorbable hemostat each available from Johnson & Johnson Wound Management, a division of Ethicon, Inc., Somerville, NJ.

Absorbable oxidized regenerated cellulose woven or knitted fabrics can be used to prepare the powder. Such fabrics, for example, are described in U.S. Pat. Nos. 4,626,253, 5,002,551 and 5,007,916.

In order to achieve adhesion prevention, the carboxyl content of the OC is from about 9% to about 18% e.g., as per United States Pharmacopeia (USP) 23-NF18. Prevention can be achieved by administration of a compound to a subject prone to develop adhesions.

In some embodiments, the carboxyl content of the OC is equal or above 12 % and equal to or below 18% as per United States Pharmacopeia (USP) 23-NF18.

Absorbable oxidized regenerated cellulose non-woven, woven or knitted fabrics can be used to prepare the powder.

Suitable oxidized regenerated cellulose fabrics include absorbable adhesion barriers such as INTERCEED absorbable adhesion barrier available from Ethicon, Inc., Somerville, N.J.

In some embodiments, the fabric is a warp knitted tricot fabric constructed of bright rayon yarn that is subsequently oxidized to include carboxyl or aldehyde moieties in amounts effective to provide the fabrics with biodegradability. The fabric is oxidized by reacting the cellulose with a solution of nitrogen dioxide in a perfluorocarbon solvent as described by F. Boardman et al. in US Patent Number 5,180,398. In one embodiment, the carboxyl content ranges from about 12% to about 18% (weight/weight). In another embodiment, the oxidized regenerated cellulose woven fabric carboxyl content (degree of oxidation) ranged from about 9.5% to about 10.5% (weight/weight).

In some embodiments, the carboxyl content of the OC powder is equal or above 12%.

In some embodiments, the carboxyl content of the OC powder is equal or above 12% and equal to or below 18% (weight/weight) as per United States Pharmacopeia (USP) 23-NF18.

Absorbable oxidized regenerated cellulose non-woven, woven or knitted fabrics may be used to prepare the powder.

Suitable oxidized regenerated cellulose fabrics for preparing the powder include absorbable adhesion barriers such as INTERCEED absorbable adhesion barrier available from Ethicon, Inc., Somerville, N.J.

In some embodiments, the fabric is a warp knitted tricot fabric constructed of bright rayon yarn that is subsequently oxidized to include carboxyl or aldehyde moieties in amounts effective to provide the fabrics with biodegradability. The fabric is oxidized by reacting the cellulose with a solution of nitrogen dioxide in a perfluorocarbon solvent as described by F. Boardman et al. in US Patent Number 5,180,398. In another embodiment, the carboxyl content (degree of oxidation) ranges from about 12% to about 18% (weight/weight). In yet another embodiment, the oxidized regenerated cellulose woven fabric carboxyl content (degree of oxidation) ranged from about 9.5% to about 10.5%.

In some embodiments, the paste is flowable at one or more temperature values selected from the group consisting of 10°C, 15°C, 20°C, 25°C, 30°C, 35°C, 37°C, and 40°C. In some embodiments, the ratio of glycerol to OC is at least about 2:1 w/w. In some embodiments, the ratio of glycerol to OC is about 6:1 or lower. In some embodiments, the paste is not flowable at one or more temperature value selected from the group consisting of 10°C, 15°C, 20°C, 25°C, 30°C, 35°C, 37°C, and 40°C. In some embodiments, the ratio of glycerol to OC is about 4:1 w/w or lower.

In some embodiments, the carboxyl content of the OC is about 12% to about 18%. In some embodiments, the composition is flowable at one or more temperature values selected from the group consisting of 10°C, 15°C, 20°C, 25°C, 30°C, 35°C, 37°C, and 40°C. In some embodiments, the ratio of glycerol to OC is at least about 2:1 w/w. In some embodiments, the ratio of glycerol to OC is about 6:1 or lower. In some embodiments, the compositions in which the carboxyl content of the OC is about 12% to about 18% are for use as an adhesion prevention material.

A method for preparing a composition comprising oxidized cellulose (OC) and glycerol, comprises the steps of:
a. combining milled OC having a water content of about 12% (w/w) or lower with glycerol at a glycerol:OC ratio of at least about 0.5:1 w/w;
b. optionally heating the composition to above room temperature; and
c. optionally further adding glycerol to the composition,
so as to obtain a composition characterized by a paste consistency at one or more temperature value selected from the group consisting of 15°C, 20°C, 25°C, 30°C, 35°C, 37°C, and 40°C.

In some embodiments of the composition, the composition has a viscosity of at least about 10% higher than that of the glycerol and lower than about 2.6X10⁹ cP (1 cP corresponds to 1 mPa·s) at one or more temperature value selected from the group consisting of 15°C, 20°C, 25°C, 30°C, 35°C, 37°C, and 40°C. In some embodiments, the ratio of glycerol to OC is between about 0.5:1 and about 6:1 w/w, respectively. In some embodiments, the OC comprises oxidized regenerated cellulose (ORC). In some embodiments, the particle size of the OC is between 10µm and 2,000µm, optionally between 50µm and 300µm. In some embodiments, the carboxyl content of the OC is about 12% to about 18%.

In a further aspect, the composition comprises oxidized cellulose (OC) and glycerol, wherein
(i) the viscosity of the composition is at least 10% higher than that of the glycerol and lower than about 2.6X10⁹ cP at one or more temperature values selected from the group consisting of: 10°C, 15°C, 20°C, 25°C, 30°C, 35°C, and 40°C;
(ii) the total water content is less than about 8% w/w; and
(iii) the composition is in the form of a paste at one or more temperature values selected from the group consisting of 10°C, 15°C, 20°C, 25°C, 30°C, 35°C, 37°C, and 40°C.

In some embodiments, the composition has a ratio of glycerol to OC of at least about 0.5:1 w/w glycerol:OC. In some embodiments, the composition has a viscosity of at least about 1,700 cP. In some embodiments, the composition has a viscosity of about 3,500 cP or lower at one or more temperature values selected from the group consisting of 10°C, 15°C, 20°C, 25°C, 30°C, 35°C, 37°C, and 40°C. In some embodiments, the composition has a viscosity of about 3,500 cP or higher at one or more temperature value selected from the group consisting of 10°C, 15°C, 20°C, 25°C, 30°C, 35°C, 37°C, and 40°C.

In some embodiments, the carboxyl content of the OC is about 12% to about 18% (weight/weight). In some embodiments, the composition has a viscosity of about 3,500 cP or lower at one or more temperature values selected from the group consisting of 10°C, 15°C, 20°C, 25°C, 30°C, 35°C, 37°C, and 40°C. The composition is for use as an adhesion prevention material.

In yet another aspect the composition is in the form of a paste comprising oxidized cellulose (OC) and glycerol, wherein:
(i) the ratio of glycerol to OC is at least about 0.5:1 w/w glycerol:OC;
(ii) the total water content is less than about 8% w/w; and
(iii) the viscosity of the composition is at least 10% higher than that of the glycerol and lower than about 2.6X10⁹ cP at one or more temperature values selected from the group consisting of 10°C, 15°C, 20°C, 25°C, 30°C, 35°C, 37°C, and 40°C.

The composition may be provided in a kit, wherein the kit comprises:
a. a container containing the composition of the invention as defined in any aspect and embodiment provided herein;
b. an applicator for applying the composition to a tissue; and
c. optionally instructions for use.

The container may be comprised in the applicator.

Disclosed is also an adhesion prevention powder, e.g., gamma radiated powder, comprising OC having a carboxyl content of from about 9% to about 18% (by weight) (e.g., 9%, 10%, 11%, 12%, 13%, 14%, 15%, 16%, 17%, or 18%, including any value and range therebetween), characterized by adhesion prevention potency of at least 120%, or at least 150% as compared to OC fabric having a similar carboxyl content. In one embodiment, the adhesion prevention gamma radiated powder comprises milled OC in aggregated form. In some embodiments, the carboxyl content of the OC powder is equal or above 9% and equal to or below 18% (by weight) as per United States Pharmacopeia (USP) 23-NF18. In some embodiments, the carboxyl content of the OC powder is equal or above 12 % and equal to or below 18% (by weight) as per United States Pharmacopeia (USP) 23-NF 18.

Absorbable oxidized regenerated cellulose non-woven, woven or knitted fabrics can be used to prepare the powder.

Suitable oxidized regenerated cellulose fabrics for preparing the powder include absorbable adhesion barriers such as INTERCEED absorbable adhesion barrier available from Ethicon, Inc., Somerville, N.J.

In some embodiments, the fabric is a warp knitted tricot fabric constructed of bright rayon yarn that is subsequently oxidized to include carboxyl or aldehyde moieties in amounts effective to provide the fabrics with biodegradability. The fabric is oxidized by reacting the cellulose with a solution of nitrogen dioxide in a perfluorocarbon solvent as described by F. Boardman et al. in US Patent Number 5,180,398. In one embodiment, the carboxyl content (degree of oxidation) ranges from about 12% to about 18% (by weight). In yet another embodiment, the oxidized regenerated cellulose woven fabric carboxyl content (degree of oxidation) ranged from about 9.5% to about 10.5% (by weight).

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.
Fig. 1 presents graphs showing the resistance to a penetrating force of a paste prepared by combining ORC and Glycerol without mixing (the ratios refer to glycerol to ORC v/w, respectively).
**Fig. 2** presents graphs showing the resistance to a penetrating force a pasted prepared by combining ORC and Glycerol with mixing (the ratios refer to glycerol to ORC v/w, respectively).
**Fig. 3** presents a graph showing examined viscosity of INTERCEED^{®}/glycerol compositions at various w/v ratios minus glycerol background at viscosity 20-22°C. Y=36.24e ^{11.113x}; ^{R2}=0.9946.
**Fig. 4** presents a graph showing calculated viscosity of INTERCEED^{®}/glycerol compositions at various w/v ratios minus glycerol background viscosity at 20-22°C.
**Fig. 5** presents graph showing calculated viscosity of INTERCEED^{®}/glycerol compositions at various w/v ratios at 20-22°C.
**Fig. 6** presents a graph showing examined viscosity of SURGICEL^{®}/glycerol compositions at various w/v ratios minus glycerol background viscosity at 20-22°C. Y=45.526e ^{8.9243x}; R²=0.9937.
**Fig. 7** presents a graph showing calculated viscosity of SURGICEL^{®}/glycerol compositions at various w/v ratios minus glycerol background viscosity at 20-22°C.
**Fig. 8** presents graph showing calculated viscosity of SURGICEL^{®}/glycerol compositions at various w/v ratios at 20-22°C.
**Fig. 9** presents graphs showing the hemostatic activity of compositions comprising SURGICEL^{®} ORC and glycerol at a ratio of about 1:1 w/v ORC:glycerol in a porcine punch biopsy. Left to right: dry ORC (milled/ground ORC), ORC (milled/ground ORC)+ glycerol, ORC(milled/ground ORC) + glycerol and CaCl₂, ORC (milled/ground ORC)+glycerol+ thrombin, gelatin paste, gelatin paste + thrombin. Error bars show standard deviation when significant.
**Fig. 10** presents graphs showing adhesion intensity in a rat cecal abrasion model treated with compositions comprising INTERCEED^{®} ORC and glycerol. Left to right: No treatment, INTERCEED^{®}, INTERCEED^{®} powder (milled/ground ORC which was compressed into small granules), ORC milled/ground ORC which was compressed into small granules):glycerol=0.33 (w/v), ORC milled/ground ORC which was compressed into small granules):glycerol=0.5 (w/v), glycerol. n=6

### DESCRIPTION OF EMBODIMENTS OF THE INVENTION

An object of the present invention is to provide a composition comprising e.g., oxidized cellulose (OC), for preparing a spreadable paste or powder for use as an adhesion prevention material, which may easily be applied to a site of need, especially in difficult to reach areas of the body.

Finding a suitable formulation to bring the OC to an appropriate consistency is not straight-forward, since the commonly used solvates or non-solvates, such as water or oils, cannot be used without compromising the active functions of the OC.

The present invention is therefore based on the surprising finding that a composition comprising oxidized cellulose (OC) and glycerol, such that the glycerol content is at least 50% by weight compared to the OC (i.e. a weight ratio of glycerol to OC of 0.5:1 or more, respectively), is in the form of a paste around room temperature, and still maintains the functional properties of the OC. Such a composition may therefore be applied to a site of need in order to obtain biological activity such as preventing adhesions.

By "applied to a site of need" it is meant to refer e.g., to a topical application of the composition at the site, e.g., at a surgical site, for example in order to prevent adhesions.

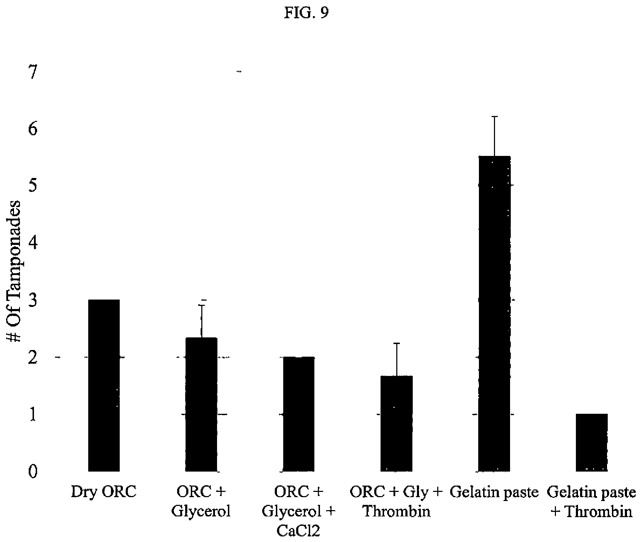

The pasty consistency of the composition enables it to be spread on the site, that is usually bleeding, such that it is not washed away with the blood, and also not spread around like a powder. As can be seen from **Fig. 9****,** the ability of a composition , comprising SURGICEL^{®} and glycerol at a v/w ratio of glycerol:OC of about 1:1 (corresponding to a w/w ratio of about 1.26:1), to stop bleeding is at least comparable to the dry OC powder (e.g. milled/ground OC or milled /ground OC in aggregated form), and much better than that of gelatin paste.

Further, as disclosed herein, when the glycerol content in the composition is about 50% by weight of the OC, it has a rather firm consistency, which allows it to be applied to bleeding sites in bones, e.g., for sealing blood vessels after surgery.

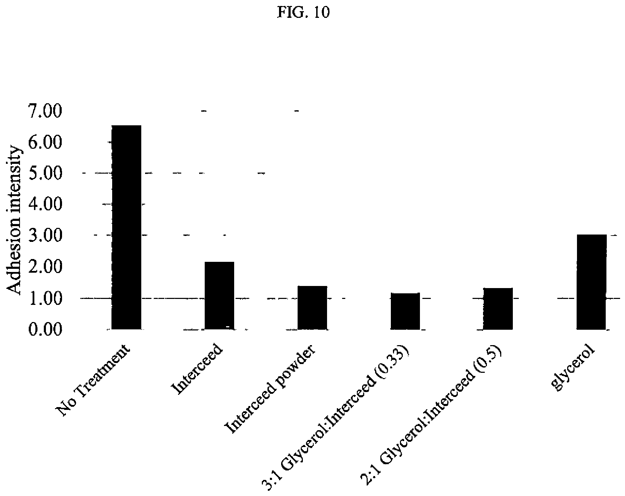

The ability of the composition comprising SURGICEL^{®} and glycerol at a v/w ratio of glycerol:OC of about 0.5:1 (corresponding to a w/w ratio of about 0.63:1), to stop bleeding upon contacting the composition with the bleeding site was tested. As can be seen from **Fig. 9****,** in this respect, the disclosed composition is better than the dry OC powder (milled/ground OC), and is much better than that of the gelatin paste without thrombin. Additionally, in a sternum bleeding model, the composition, comprising SURGICEL^{®} and glycerol at a v/w ratio of glycerol:OC of about 0.5:1, was capable of preventing or reducing bleeding even after scraping the composition from the bleeding site (data not shown). Regarding adhesion prevention function, reference is made to **Fig. 10****,** which shows a comparison of adhesion prevention using 1-INTERCEED^{®} fabric, 2- milled/ground INTERCEED which was compressed into small granules (interceed powder) and 3- interceed powder with glycerol at v/w ratios of glycerol:OC of 3:1 or 2:1 (corresponding to w/w ratios of about 3.78:1 or 2.52:1, respectively). It was found that interceed powder and interceed powder with glycerol have adhesion prevention activity better than or comparable to INTERCEED^{®} sheet or glycerol alone. It is noteworthy that gamma radiated interceed aggregated powder exhibited adhesion prevention potency of about 135% as compared to INTERCEED ^{®} fabric.

Interceed powder in the form of aggregates is also named herein: milled /ground interceed in aggregated form, milled/ground interceed which was compressed into small granules, compacted interceed powder, interceed compacted in the form of aggregate.

Interceed powder in the form of aggregates can be generated by the process described in US9539358 examples. ORC the base material is INTERCEED^{®} sheets (Ethicon) instead of SURGICEL^{®} sheets (Ethicon). Interceed powder in the form of aggregates can be subjected to 20-45 kilogray of gamma radiation (e.g. By Sorvan radiation ltd) to provide sterility.

As used herein, and unless stated otherwise, the terms "by weight", "w/w", "weight percent", or "wt. %", which are used herein interchangeably describe the concentration of a particular substance out of the total weight of the corresponding mixture, solution, formulation or composition. It is noted that the ratios indicated in the examples are in v/w of glycerol: ORC. Since the density of glycerol is 1.26 gr/ml, a ratio of 1:1 glycerol:ORC v/w corresponds to a ratio of 1.26:1 glycerol:ORC w/w.

As used herein, the term "bleeding" refers to extravasation of blood from any component of the circulatory system. A "bleeding" thus encompasses unwanted, uncontrolled and often excessive bleeding in connection with surgery, trauma, or other forms of tissue damage, as well as unwanted bleedings in patients having bleeding disorders.

As used herein, the term "adhesion" or "tissue adhesion" refers to connection of tissues not normally connected. For example, adhesions can occur as post-operative complication.

As used herein, the terms "controlling", "preventing", or "reducing", which may be used herein interchangeably in the context of the bleeding, including any grammatical inflection thereof, indicate that the rate of the blood extravagated is essentially nullified or is reduced by 10 %, at least 20 %, at least 30 %, at least 40 %, at least 50 %, at least 60 %, at least 70 %, at least 80 %, at least 90 %, or even by 100 %, of the initial rate of bleeding, compared to situation lacking the contact of the disclosed composition in/on the bleeding site. Methods for determining a level of appearance of bleeding are known in the art.

Further, in some embodiments, the terms "controlling", "preventing" or "reducing", in the context of the bleeding are also meant to encompass at least partially sealing blood vessels at the bleeding site either in soft tissues or bone tissues.

As used herein, the terms "controlling", "preventing", or "reducing", which may be used herein interchangeably in the context of the tissue adhesion, including any grammatical inflection thereof, indicate that the formation of tissue adhesion is completely or partially prevented, or the severity of the adhesion is lower, for example according to the adhesion evaluation scheme according to Poehnert et al., 2015, International journal of medical sciences 12(1):1-6, described in the examples section.

Accordingly, in one aspect, the composition comprises oxidized cellulose (OC) and glycerol, wherein: the ratio of glycerol to OC is at least about 0.5:1 w/w glycerol:OC (i.e. more than "0.5" glycerol in the above-mentioned ratio); the total water content being less than about 8%, by weight; and the composition is in the form of a paste at around room temperature.

By "around room temperature" it is meant to refer to at least one temperature value within the range of 10°C to 40°C, or 15°C to 37°C. e.g., 10°C, 15°C, 20°C, 25°C, 30°C, 35°C, 37°C, or 40°C, including any value therebetween.

Accordingly, in some embodiments, the disclosed composition is in the form of a paste at one or more temperature value selected from the group consisting of 10°C, 15°C, 20°C, 25°C, 30°C, 35°C, 37°C, and 40°C.

In some embodiments, the composition has a viscosity of at least 10% higher than that of glycerol at one or more temperature values selected from the group consisting of 10°C, 15°C, 20°C, 25°C, 30°C, 35°C, 37°C, and 40°C.

In some embodiments, the composition has a viscosity of at least about 5%, 6%, 7%, 8%, 9%, 10%, 11%, 12%, 13%, 14%, 15%, 16%, 17%, 18%, 19%, or 20% higher than that of glycerol at one or more temperature values selected from the group consisting of 10°C, 15°C, 20°C, 25°C, 30°C, 35°C, 37°C, and 40°C.

In some embodiments, the compositions of the invention have a viscosity lower than about 2.6X10⁹ centipoise (cP) at one or more temperature values selected from the group consisting of 15°C, 20°C, 25°C, 30°C, 35°C, 37°C, and 40°C.

In some embodiments, the compositions of the invention have a viscosity lower than about 2.0X10⁴ centipoise (cP) at one or more temperature values selected from the group consisting of 15°C, 20°C, 25°C, 30°C, 35°C, 37°C, and 40°C.

In some embodiments, the compositions of the invention have a viscosity lower than about 5X10⁹, 2.6X10⁹, 1X10⁹, 5X10⁸, 1X10⁸, 5X10⁷, 1X10⁷, 5X10⁶, 1X10⁶, 5X10⁵, 1X10⁵, 5X10⁴, 2X10⁴, or 1X10⁴ cP at one or more temperature values selected from the group consisting of 10°C, 15°C, 20°C, 25°C, 30°C, 35°C, 37°C, and 40°C.

The viscosity of the composition is measured by any suitable method, for example by using a viscometer, as elaborated in more detail below and in **Example 2.**

In some embodiments, the ratio of glycerol:OC is between about 0.5:1 and about 6:1 w/w.

In some embodiments, the ratio of glycerol:OC is between 0.5:1 and 5:1 w/w; between 0.5:1 and 4:1 w/w; between 0.5:1 and 3:1 w/w; between 0.5:1 and 2:1 w/w; between 0.5:1 and 1:1 w/w; or between 0.5:1 and 0.9:1 w/w.

In some embodiments, the ratio of glycerol:OC is between 0.9:1 and 6:1 w/w, respectively; between 1:1 and 6:1 w/w; between 1.5:1 and 6:1 w/w; between 0.9:1 and 5:1 w/w; between 0.9:1 and 4:1 w/w; between 1:1 and 4:1 w/w; between 1.5:1 and 4:1 w/w; or between 1.5:1 and 3:1 w/w.

In some embodiments, the weight ratio of glycerol to OC is about 0.5:1, 0.6:1, 0.7:1, 0.8:1, 0.9:1, 1:1, 1.1:1, 1.2:1, 1.3:1, 1.4:1, 1.5:1, 2:1, 2.5:1, 3:1, 3.5:1, 4:1, 4.5:1, 5:1, 5.5:1, or 6:1 including any ratio therebetween.

In some embodiments, the weight of the glycerol is more than 50% compared to the weight of the OC. In some embodiments, the weight of the glycerol is more than 60%, 70%, 80%, 90%, or more than 100% compared to the weight of the OC.

The term "paste" as used herein, relates to the consistency of the composition at at-least one temperature around the room temperature, and defines a fluid mixture of solid particles.

Non-limiting exemplary solid particles comprise ORC fiber and/or granules.

Non-limiting exemplary non-solvent liquid comprises glycerol. The paste may have a malleable, putty-like consistency, such as wax, toothpaste or ointments. The paste may behave as a solid until a force is applied, at which point it could flow like a fluid. Typically, but not exclusively, the paste conforms, by applying manual pressure or by gravity, to irregular surfaces upon application. Typically, but not exclusively, pastes comprise a suspension of a material in a surrounding fluid.

The term "non-solvent" as used herein refers to a liquid, or a mixture of liquids, which is incapable of dissolving any appreciable concentration (e.g., a concentration less than about 5 %, less than about 2 %, less than about 1 %, less than about 0.5 %, less than about 0.2 %, or less than about 0.1 % at around the room temperature) of a particle of interest, e.g., OC.

In some embodiments, the individual particles adhere together in the paste, like grains of earth in mud, forming a disordered, glassy or amorphous structure, and giving pastes their solid-like character. Some of the paste unique properties may be derived from the particles adherence, demonstrating properties similar to that of a fragile matter.

A paste is distinct from gel, since the solids in the gel are typically dissolved within a liquid. A paste is further distinct from a jelly, that changes its properties once broken.

The term "paste" according to the present disclosure may also include a slurry. A slurry may functionally be regarded as a thin, watery paste. A paste according to the present disclosure may also include pores comprising of an expandable gas, such as air. Accordingly, the composition is a paste, or is in a paste (or pasty) consistency at around room temperature.

A further distinction in the paste consistency of the compositions of the invention is made between a "flowable" and a "non-flowable" paste. The term "non-flowable" (or "not flowable") paste is also referred to herein as "putty". As used herein, the term "flowable" in the context of paste relates to a more fluid consistency at around the room temperature, which may still flow after application of the composition in/on the bleeding site. The terms "non-flowable" or "putty" refer to a doughier consistency at 37 °C, which takes longer to settle, and has a better shape retention than a "flowable" paste.

In some embodiments, the composition is homogeneous.

As used herein, by "homogeneous" it is meant to refer to a uniform composition and texture throughout.

In order to prepare a spreadable agent, e.g., in the form of paste, the appropriate solvate or non-solvate has to be identified. Since oxidized regenerated cellulose (ORC) disintegrates in water, a non-aqueous thickening agent is preferred. Although hydrophobic agents such as olive oil, soybean oil, fish oil and copra oil may first be conceived of, the inventors have found that because of the aqueous environment at the bleeding site, using oil results in the composition floating on top of the bleeding, thereby being ineffective in sealing the bleeding site. Further, the inventors have surprisingly realized that adding glycerol to the OC provided the composition with the desired consistency while not affecting its activity.

Although the consistency of the composition as "paste" is defined above, alternatively, or additionally, the composition may also be defined in terms of resistance under certain conditions, as detailed hereinbelow.

In accordance with the present application, a tensile machine, such as an LF Plus Tensile Machine (Lloyd Instruments) may be used for monitoring the resistance of the composition to a metallic cylindrical probe with a flat edge.

In exemplary embodiments, the Tensile Machine settings are adjusted to monitor a probe having a 1.27 cm diameter at a speed of 30 mm/min from a defined preload of 0.1 N at 8 mm penetration. The glycerol/ORC compositions of the invention may be incubated for an overnight at 60-80 °C and may be either mixed or not mixed prior to testing. All compositions have a resistance of under 20 N (under 10 N for the mixed compositions) when tested at around the room temperature.

Accordingly, in some embodiments, the composition has a resistance lower than about 20 N as measured in a tensile machine adjusted to monitor a probe having a 1.27 cm diameter at a speed of 30 mm/min from a defined preload of 0.1 N at 8 mm penetration at one or more temperature values selected from the group consisting of 10°C, 15°C, 20°C, 25°C, 30°C, 35°C, 37°C, and 40°C.

It is noted that the same conditions are used throughout the application to measure the resistance of the composition, and therefore, these conditions apply for all resistance measurements even if not explicitly stated.

In some embodiments, the composition has a resistance of less than 19 N, less than 18 N, or less than 17 N. In some embodiments, the composition has a resistance of between 1 N and 20 N, between 1 N and 19 N, between 1 N and 18 N, or between 1 N and 17 N.

In some embodiments, the composition has a resistance of less than 16 N, less than 15 N, less than 14 N, less than 13 N, less than 12 N, less than 11 N, less than 10 N, less than 9 N, less than 8 N, or less than 7 N. In some such embodiments, the paste composition as described herein is in the form of non-flowable paste (putty) or flowable paste.

In some embodiments, the composition has a resistance of less than 6 N, less than 5 N, less than 4 N, or less than 3 N. In some embodiments, the composition has a resistance of between 1 N and 7 N, between 1 N and 6 N, between 1 N and 5 N, between 1 N and 4 N, or between 1 N and 3 N. In some such embodiments, the paste composition as described herein is in the form of non-flowable paste (putty) or flowable paste.

Hereinthroughout, the term "resistance" refers to "resistance to penetration" or to the force required to penetrate the disclosed composition as measured according to the so-called "Bloom test".

Exemplary methods for determining the resistance to penetration are described hereinbelow.

In some embodiments, the composition has a resistance higher than about 1 N as measured in a tensile machine adjusted to monitor a probe having a 1.27 cm diameter at a speed of 30 mm/min from a defined preload of 0.1 N at 8 mm penetration at one or more temperature values selected from the group consisting of 10°C, 15°C, 20°C, 25°C, 30°C, 35°C, 37°C, and 40°C.

In some embodiments, the composition has a resistance higher than 2 N, higher than 3 N, higher than 4 N, higher than 5 N, higher than 6 N, higher than 7 N, higher than 8 N, higher than 9 N, higher than 10 N, higher than 11 N, higher than 12 N, higher than 13 N, higher than 14 N, higher than 15 N, or higher than 16 N. In some embodiments, the composition has a resistance of between 2 N and 20 N, between 3 N and 20 N, between 4 N and 20 N, between 5 N and 20 N, between 6 N and 20 N, between 7 N and 20 N, between 8 N and 20 N, between 9 N and 20 N, between 10 N and 20 N, between 11 N and 20 N, between 12 N and 20 N, between 13 N and 20 N, between 14 N and 20 N, between 15 N and 20 N, or between 16 N and 20 N. In some embodiments, the composition described herein is in the form of a putty.

It is appreciated that for flowable compositions, when the OC is already saturated with glycerol, additional glycerol may be added and possibly not affect the consistency of the composition. Accordingly, ratios of glycerol to OC which are above saturation level are intended to be included in the compositions of the invention.

It is further appreciated that different consistencies may be obtained for a certain ratio of glycerol to OC, depending on whether the composition is used immediately following combining the OC and the glycerol or not.

Further, in some embodiments, if the composition is not used immediately following combining the OC and the glycerol, the consistency of the composition depends on the temperature and/or the length of time of the incubation.

Without being bound by any particular theory or mechanism, it appears that incubation for longer periods of time and at elevated temperatures causes more glycerol to be absorbed in the OC, which, in turn, causes the composition to be drier, or more solid.

In some embodiments, incubating at elevated temperatures or for longer times may require adding more glycerol to obtain pasty consistency. The ratio of glycerol to OC is required to obtain a certain consistency may depend on the OC structure.

By "elevated temperatures" it is meant to refer to temperatures higher than 25°C, higher than 30°C, higher than 35°C, or higher than 40°C, and up to e.g., 70°C, 80°C, 90°C, or 100°C.

Since the function of the compositions of the invention depends on their consistency, i.e., being a paste at around the room temperature as defined above, any ratio of glycerol to OC which results in such a consistency is intended to be included in the invention.

The term "oxidized cellulose" (or "OC") refers to a cellulose derivative in which at least some of the primary alcohol groups, e.g., on the carbon 6 of the anhydroglucose unit is oxidized to a carboxylic acid, and is optionally functionalized.

OC may be produced by applying an oxidizing agent on cellulose. The oxidizing agent may be selected from, without being limited thereto, chlorine, hydrogen peroxide, peracetic acid, chlorine dioxide, nitrogen dioxide, persulfates, permanganate, dichromate-sulfuric acid, hypochlorous acid, hypohalites, periodates, or any combination thereof, and/or a variety of metal catalysts. Oxidized cellulose may contain carboxylic acid, aldehyde, and/or ketone groups, instead of, or in addition to the original hydroxyl groups of the starting material, cellulose, depending on the nature of the oxidant and reaction conditions.

The OC used in the compositions of the invention is in the form of a powder (also referred to as milled/ground OC or milled /ground OC in aggregated form). The milled/ground OC may be prepared by various methods including from existing products, and some non-limiting examples of such products are described below. As some of the existing products are in the form of a fabric, the OC powder may be prepared by grinding or milling the fabric to obtain a powder. For example, milled OC (or ORC) may be obtained by reducing the size of an OC sheet, such as a SURGICEL^{®} or an INTERCEED^{®} sheet, by milling, as described in US9539358.

US9539358 discloses preparation of compacted Powders comprising ORC-Ball-Milled powders (BMP)

Several pieces of 4"×4" pre-trimmed non-sterile SURGICEL fabric (ETHICON, Inc., Lot #7A8654), can be vacuumed dried for 24 hours prior to milling. 6-gram samples can be mixed with 12 high-density ZrO₂ balls (20 mm in diameter; Glen Mills Inc., Clifton, N.J., USA) and sealed in a 250 mL grinding jar. The jar can be clamped into the latching brackets and then counterbalanced on the mill (planetary ball mill PM100; Retsch, Inc., Newtown, Pa., USA). Milling can be carried out at 300 rpm for 10 min. The milled powder then can be dried in a vacuum oven (Fisher Scientific Model 280A Isotemp vacuum oven) with a vacuum pump (LabCare America Pump PV-35) at 65° C. for 2.5 h. The milled powder may be finally stored in a nitrogen box. Similar method as above can be used to prepare powder with ORC-based SURGICEL^{®}NU-KNIT^{®} absorbable hemostat. Roller-Compacted ORC powder can be prepared by using ORC shredded through a Fitz Mill equipped with a screen mesh 1726-150. The shredded ORC powders can be fed into a roller compactor (WP 120x 40V, #900-0071, Alexanderwerk, Inc, PA) and compacted as described in US9539358.

In exemplary embodiments, OC has been oxidized to contain carboxyl moieties in amounts effective to provide biodegradability.

U.S. Pat. No. 3,364,200 discloses the preparation of carboxylic-oxidized cellulose with an oxidizing agent such as dinitrogen tetroxide in a Freon medium. U.S. Pat. No. 5,180,398 discloses the preparation of carboxylic-oxidized cellulose with an oxidizing agent such as nitrogen dioxide in a per-fluorocarbon solvent. After oxidation by either method, the fabric may be thoroughly washed with a solvent such as carbon tetrachloride, followed by aqueous solution of 50 percent isopropyl alcohol (IPA), and finally with 99% IPA. Prior to oxidation, the fabric can be constructed in the desired woven or nonwoven construct.

Typically, hemostats that are compatible with acid-sensitive species comprise fabric substrates prepared from a biocompatible, aldehyde-oxidized polysaccharide. In such exemplary hemostats, the polysaccharide contains an amount of aldehyde moieties effective to render the modified polysaccharide biodegradable, meaning that the polysaccharide is degradable by the body into components that are either resorbable by the body, or that can be passed readily by the body. More particularly, the biodegraded components do not elicit permanent chronic foreign body reaction when they are absorbed by the body, such that substantially no permanent trace or residual of the component is retained at the implantation site.

In certain embodiments of the present invention, the OC comprises particles prepared from a biocompatible, biodegradable, aldehyde-oxidized regenerated cellulose. In some embodiments, the aldehyde-oxidized regenerated cellulose is one comprising repeating units of Structure II in U.S. Pat. No. 8,709,463. In some embodiments, oxidized regenerated cellulose is used to prepare adhesion prevention material. Typically, regenerated cellulose is preferred due to its higher degree of uniformity versus cellulose that has not been regenerated. Regenerated cellulose and a detailed description of how to make regenerated oxidized cellulose is set forth in U.S. Pat. No. 3,364,200 and U.S. Pat. No. 5,180,398,

Accordingly, in some embodiments, the OC comprises oxidized regenerated cellulose (ORC). Examples for OC-based products that are either in aggregated form or may be ground or milled and therefore may be utilized to prepare particles of the composition include, but are not limited to, INTERCEED^{®} absorbable adhesion barrier, SURGICEL^{®} Original absorbable hemostat, SURGICEL^{®} NU-KNIT^{®} absorbable hemostat, SURGICEL^{®} FIBRILLAR^{™} absorbable hemostat, SURGICEL^{®} SNoW^{™} absorbable hemostat and SURGICEL^{®} Powder absorbable hemostat, GelitaCel^{®} resorbable cellulose surgical dressing from Gelita Medical BV, Amsterdam, The Netherlands.

SURGICEL^{®} Powder absorbable hemostat is a powder that comprises aggregate of small ORC fiber fragments that may spread across a large surface area and form a durable clot that will not wash away or rebleed when irrigated.

It is appreciated that while the usual source for OC is plant material, OC may also be derived from a bacterial source. In some embodiments, the OC is derived from a plant source.

In some embodiments the cellulose for use with the present invention does not include carboxymethyl cellulose (CMC).

The compositions of the invention may be non-aqueous compositions, which means that the main liquid in the compositions is not water and the compositions have a very low water content, or no water at all.

In some embodiments, the water content of the composition is lower than about 7% w/w. In some embodiments, the total water content of the composition is lower than about 6%, 5%, 4%, 3%, 2%, 1%, 0.5%, 0.1%, or 0.01% w/w. In some embodiments, the composition is substantially devoid of water. In some embodiments, the composition does not contain water.

In some embodiments, the composition does not further comprise a solvent. In some embodiments, the composition does not further comprise an organic solvent, such as, for example, ethanol.

In some embodiments, the composition consists essentially of OC and glycerol. The composition of the invention comprises OC in the form of a powder (milled OC). As indicated above, various cellulose-based materials may be ground or milled to obtain a powder which may be used to prepare the composition of the present invention.

The cellulose-based material, e.g., cellulose-based fabric, can be milled to obtain fibers that have a size distribution of D90 of less than 350 µm and of D50 of less than 167 µm. If desired, the milling step can be repeated to obtain a size distribution of D90 of less than 177 µm, and D50 of less than 95 µm.

In one embodiment, the fibers for making the composition are prepared by milling a cellulosic source material; the milling step may be preceded by forming material pieces by slitting and cutting the cellulosic source material. In this embodiment the milling step may be a two-part process with the second part performed in an air classifier wherein the second part can be repeated three times. After a first pass (time) in the air classifier, the resulting "long fibers" have a size distribution of D90 of less than 350 µm and D50 of less than 167 µm. After 3 passes (3 times) in the air classifier the resulting fine ORC fibers have a size distribution of D90 of less than 177 µm and D50 of less than 95 µm.

In one embodiment of the invention, the "fine or short" cellulose-based fibers in the composition have a size distribution of D90 of less than 177 µm, and D50 of less than 95 µm. The cellulose-based material may be mixed or supplemented with the compounds before, during and/or after the milling steps.

The terms "D50", D70" and "D90" refers to 50%, 70%, and 90%, respectively (by numbers or volume), of the particles having a size that is less than or equal to the value.

In one embodiment, the powder compositions according to the invention comprising the fibers and the compounds are compacted in the form of aggregates, optionally using steps of drying, milling/grounding and sieving as described in US10034957B2. The sieve used defines the particle size of the powder.

In some embodiments, the composition of the invention is prepared from OC in the form of aggregates. The term "aggregate" describes a particle formed from assembled components.

Aggregates may be optionally made by one of the following: including a step of humidifying the powder composition; compacting, e.g., by roller and/or slugging the powder to form aggregates; dehumidifying; milling; sieving the aggregates; and optionally dosing the resulting aggregates into a storage container or into a delivery device.

In some embodiments, the particle size of the OC is between 10 µm and 2,000 µm. In some embodiments, the particle size of the OC is between 50 µm and 1,500 µm, between 100 µm and 1,000 µm, between 100 µm and 500 µm, between 100 µm and 300 µm, between 50 µm and 1,000 µm, between 50 µm and 500 µm, or between 50 µm and 300 µm.

Calcium is an important element in the clotting cascade. It is needed for activation of factor XIII into factor XIIIa, which cross-links and stabilizes fibrin to generate an insoluble clot.

In some embodiments, the composition may further comprise at least one biologically active agent. Non-limiting biologically active agents that may be included in the composition include calcium, as well as therapeutic agents such as antibiotics, anti-inflammatory agents, growth factors, or clotting factors. For example, the composition may further comprise fibrinogen or thrombin.

In some embodiments, the composition may further comprise thrombin.

In some embodiments, the composition further comprises calcium. Calcium used with the invention may be in the form of calcium chloride salt. Alternatively, other salts may be used, such as, calcium acetate and/or calcium citrate.

In some embodiments, the composition may comprise more than one biologically active agent, for example, calcium and thrombin.

As used herein, "thrombin" denotes an activated enzyme which results from the proteolytic cleavage of prothrombin (factor II). Thrombin may be produced by a variety of methods of production known in the art, and includes, but is not limited to, recombinant thrombin and plasma derived thrombin.

Human thrombin is a 295 amino acid protein composed of two polypeptide chains joined by a disulfide bond. Both human and non-human (e.g., bovine) thrombins may be used within the scope of the present disclosure.

The composition may further include one or more of the following excipients selected from, without being limited thereto, calcium, albumin, saccharides, saccharides derivatives, polyol/s, acetate, citrate, amino acids, polyethylene glycol, and sodium chloride.

In some embodiments, the calcium source is calcium chloride e.g., in a range of 40-60 mM.

The albumin may be in a range of 0.05-1% (w/v) or in a range of 0.5-1% (w/w). The saccharides source may be saccharose and may be in a 5 g/l concentration.

In some embodiments, the saccharides derivatives source is gluconic acid. In some embodiments, the polyol/s source is mannitol e.g. (w/w) concentration of 2%. In some embodiments, the acetate source is sodium acetate and may be present e.g., at a concentration of 10 mM. In some embodiments, the citrate source can be sodium citrate.

In some embodiments, the amino acids comprise histidine and may be present at a concentration of 10 mM concentration. In some embodiments, the polyethylene glycol (PEG) source is PEG-3350 and may be present e.g., at a concentration of 0.03%, by weight. In some embodiments, the sodium chloride is present at a concentration ranging from 50 to 175 mM.

"PEG 3350" denotes a PEG compound with an average molecular weight of 3350 Daltons.

Accordingly, in some embodiments, the composition of the invention further comprises one or more excipients selected from the group consisting of sodium chloride, mannitol, albumin, and sodium acetate.

In some embodiments, the only polyol in the composition is glycerol. In some embodiments, the only polyols in the composition are glycerol and mannitol.

As mentioned and defined above, the consistency of the composition may further be divided into a more fluid paste, referred to herein as "flowable", and a doughier paste, referred to herein as "non-flowable" (or "not flowable").

The consistency of the composition is generally determined by the ratio of glycerol to OC. Typically, but not exclusively, the higher the amount of glycerol - the more fluid (flowable) the composition is, and the lower the amount of glycerol - the doughier (or more solid) and less flowable the composition is.

As also explained above, the consistency of the composition may be further affected by factors such as the time and temperature of incubation following combining the glycerol and the OC, and by the OC source, and therefore the ratio of glycerol to OC does not *a priori* exactly define the consistency of the composition.

Accordingly, in some embodiments, the composition is flowable at at-least one temperature around room temperature, such as at one or more temperature values selected from the group consisting of 10°C, 15°C, 20°C, 25°C, 30°C, 35°C, 37°C, and 40°C. This composition is referred to hereinbelow as the "flowable" composition.

Accordingly, in some embodiments, the composition is non-flowable at at-least one temperature around room temperature, such as at one or more temperature values selected from the group consisting of 10°C, 15°C, 20°C, 25°C, 30°C, 35°C, 37°C, and 40°C. This composition is referred to hereinbelow as the "non-flowable" or "not flowable" composition.

In some embodiments, the flowable composition has a resistance of 16 N or lower. In some embodiments, the flowable composition has a resistance of less than 15 N, less than 14 N, less than 13 N, less than 12 N, less than 11 N, less than 10 N, less than 9 N, less than 8 N, or less than 7 N, less than 6 N, less than 5 N, less than 4 N, or less than 3 N. In some embodiments, the flowable composition has a resistance of between 1 N and 16 N, between 1 N and 15 N, between 1 N and 14 N, between 1 N and 13 N, between 1 N and 12 N, between 1 N and 11 N, between 1 N and 10 N, between 1 N and 9 N, between 1 N and 78 N, between 1 N and 7 N, 1 N and 6 N, between 1N and 5 N, between 1 N and 4 N, or between IN and 3N.

In some embodiments, the non-flowable composition has a resistance higher than 2 N, higher than 3 N, higher than 4 N, higher than 5 N, higher than 6 N, higher than 7 N, or higher than 8 N, higher than 9 N, higher than 10 N, higher than 11 N, higher than 12 N, higher than 13 N, higher than 14 N, higher than 15 N, or higher than 16 N.

In some embodiments, the non-flowable composition has a resistance of between 2 N and 20 N, between 3 N and 20 N, between 4 N and 20 N, between 5 N and 20 N, between 6 N and 20 N, between 7 N and 20 N, between 8 N and 20 N, between 9 N and 20 N, between 10 N and 20 N, between 11 N and 20 N, between 12 N and 20 N, between 13 N and 20 N, between 14 N and 20 N, between 15 N and 20 N, or between 16 N and 20 N.

In some embodiments, the ratio of glycerol to OC in the flowable composition is about 2:1 w/w or higher. In some embodiments, the ratio of glycerol to OC in the flowable composition is about 2.5:1, 3:1, 3.15:1, 3.5:1, 3.78:1, 4:1, or higher. In some embodiments, the ratio of glycerol to OC in the flowable composition is about 6:1 or lower. In some embodiments, the ratio of glycerol to OC in the flowable composition is about 5.5:1, about 5:1, about 4.5:1, about 4:1 or lower. In some embodiments, the ratio of glycerol to OC in the flowable composition is between about 2:1 and about 6:1.

In some embodiments, the ratio of glycerol to OC in the non-flowable composition is between about 0.5:1 and 4:1 w/w. In some embodiments, the ratio of glycerol to OC in the non-flowable composition is between about 0.5:1 and 3.5:1 w/w, between 0.5:1 and 3:1.

In some embodiments, the ratio of glycerol to OC in the non-flowable composition is about 4:1 w/w or lower. In some embodiments, the ratio of glycerol to OC in the non-flowable composition is about 3.78:1, 3.5:1, 3.15:1, 3:1, 2.5:1, 2:1, 1.5:1, 1:1 w/w, or lower.

In some embodiments, the ratio of glycerol to OC in the non-flowable composition is 0.5:1, 1:1, 1.5:1, 2:1, 2.5:1, 3:1, 3.15:1, 3.5:1, 3.78:1, or 4:1 w/w, including any value and range therebetween.

In some embodiments, the composition is capable of being passed through a needleless syringe. Such a syringe may have an outlet orifice diameter of 0.9-1.2 mm. Accordingly, in some embodiments, the flowable composition is capable of being manually passed through an orifice of at least about 0.9 mm and above.

The term "manually" as used herein defines the force applied in order to pass the composition through the syringe as a reasonable force that may be applied by the average human, for example by the surgeon or the nurse.

As indicated above, the degree of oxidation of the OC is important to its functional properties such as biocompatibility and bioabsorbability. Products including various degrees of OC oxidation exist, such as a surgical hemostat in which carboxylic acid groups are present at a concentration of 18-21 % (by weight) of the oxidized cellulose. On the other hand, OC with a lower concentration of carboxylic acid groups, such as 12%-18% has adhesion prevention properties.

As used herein with reference to OC, the terms "oxidation level", "degree of oxidation", "carboxyl content" and "carboxylation level" are interchangeable, and may be determined per United States Pharmacopeia (USP) 23-NF18.

In some embodiments, the carboxyl content of the OC is about 12% to about 18% (w/w). In some embodiments, the carboxyl content of the OC is 12-17% (w/w). In some embodiments, the carboxyl content of the OC is 12-16% (w/w).

In some embodiments, the carboxyl content of the OC is about 12%, 13%, 14%, 15%, 16%, 17%, or 18% (w/w) including any value and range therebetween.

Control of bleeding is needed in various situations including treatment of wounds, or during surgical procedures, such as, for example, neurosurgery, abdominal surgery, cardiovascular surgery, thoracic surgery, head and neck surgery, pelvic surgery and skin and subcutaneous tissue procedures.

The non-flowable composition is defined, in some embodiments, as "non-flowable" at 37°C. In some embodiments, a "non-flowable" composition is also non-flowable at room temperature.

The term "soft tissues" as used herein relates to body tissue that is not hardened or calcified. This term especially relates to soft tissues that are vascularized and therefore may be a source of bleeding. Examples for such tissues include but are not limited to connective tissue (such as tendons, ligaments, fascia, skin, fibrous tissues, fat, and synovial membranes), muscles, and internal organs. In general, soft tissues are meant to exclude bone tissue.

The composition of the invention, having an OC with a carboxyl content of about 12% to about 18% is useful as an adhesion prevention composition, for preventing formation of adhesions

In some embodiments, the paste in which the carboxyl content of the OC is about 12% to about 18%, is flowable at one or more temperature value selected from the group consisting of 10°C, 15°C, 20°C, 25°C, 30°C, 35°C, 37°C, and 40°C. In some embodiments, the ratio of glycerol to OC in the flowable paste is at least about 2:1 w/w. In some embodiments, the ratio of glycerol to OC in the flowable paste is about 6:1 w/w or lower.

In some embodiments, a fabric for preparing the powder is a warp knitted tricot fabric constructed of bright rayon yarn that is subsequently oxidized to include carboxyl or aldehyde moieties in amounts effective to provide the fabrics with biodegradability. The fabric is oxidized by reacting the cellulose with a solution of nitrogen dioxide in a perfluorocarbon solvent as described by F. Boardman et al. in US Patent Number 5,180,398.

In one embodiment, OC/ORC with a carboxyl content (degree of oxidation) ranging from about 12% to about 18% are used in adhesion prevention. In yet another embodiment, the oxidized regenerated cellulose with a carboxyl content (degree of oxidation) ranging from about 9.5% to about 10.5% are in adhesion prevention.

In some embodiments, the flowable or the non-flowable compositions of the invention in which the carboxyl content of the OC is about 12% to about 18% are used as an adhesion prevention material, for reducing or preventing adhesions. In some embodiments, the flowable compositions of the invention in which the carboxyl content of the OC is about 12% to about 18% are used as an adhesion prevention material, for reducing or preventing adhesions.

In some embodiments, the compositions of the invention are further treated to obtain a low bioburden, by methods known in the art such as heat treatment, radiation treatment, filtration or chemical treatment, e.g., gamma radiation, filtration using a pore size of 0.22 µm or lower, heat sterilization and aseptic field.

In a different aspect, the present invention provides an adhesion prevention composition comprising oxidized cellulose (OC) and glycerol, wherein the ratio of glycerol to OC is at least about 0.5:1 w/w, the carboxyl content of the OC is about 12% to about 18 %, the total water content is about 8% w/w or lower, and the composition is in a paste around room temperature.

A method for preparing a composition comprises oxidized cellulose (OC) and glycerol in the form of a paste comprising the steps of: combining milled OC having a water content of about 12% or lower (e.g., 11%, 10%, 9%, 8% 7% or less by weight) with glycerol at a ratio of glycerol to OC about 0.5:1 w/w or higher at about 25°C (within room temperature); optionally heating the composition to above room temperature; and optionally further adding glycerol to the composition, so as to obtain a composition characterized by a paste consistency at one or more temperature value selected from the group consisting of 15°C, 20°C, 25°C, 30°C, 35°C, 37°C, and 40°C.

Heating the composition to above room temperature may be followed by cooling the composition to a desired temperature, e.g., around the room temperature.

Without being bound by any particular theory or mechanism, it is assumed that the heating step allows to speed up stabilization of the composition, e.g., by at least 10%, 20%, 30%, 40%, 50%, 60%, 70%, 80%, 90%, 100%, 200%, or more.

Accordingly, the composition may be heated to a certain elevated temperature, which is above room temperature and then cooled down to the desired temperature. The desired temperature may be around the room temperature.

As used herein, the term "heating" means increasing the temperature of the substance by applying heat.

The composition may be heated to 37 °C. The composition may be heated to 40°C, 45°C, 50°C, 55°C, 60°C, 65°C, 70°C, 75°C, 80°C, 85°C, 90°C, 95°C, or 100°C, including any value and range therebetween, or, in some embodiments, to above 100°C, prior to cooling.

The composition may be heated until the glycerol is absorbed in the OC prior to cooling. The composition may be heated until the glycerol is fully absorbed in the OC prior to cooling.

The term "absorbed" as used herein refers to the physical state in which the glycerol molecule is distributed throughout the body of the OC.

The composition may be heated for between 5 minutes and 24 hours prior to cooling. The composition may be heated for between 10 minutes and 10 hours prior to cooling. The composition may be heated for between 30 minutes and 5 hours prior to cooling.

The time needed to heat the composition varies depending on the temperature. Accordingly, for example, the composition may be placed at about 100°C for about 5 - about 10 minutes. Alternatively, the composition may be placed at about 60°C for about 30 minutes to about 5 hours. Another example is that the composition may be placed at about 40°C for about 1 hour - about 24 hours. The composition may be placed at 60-80 °C for an overnight.

It is known that heating the composition of OC with glycerol shortens the time of stabilization of the composition, which is the time it takes for it to reach its final form. The higher the temperature, the less time it takes for the composition to stabilize.

The combining of OC and glycerol may be done at the already elevated temperature.

The OC and the glycerol may be combined or mixed by any suitable method.

Therefore, additional glycerol may be added to the composition until the desired consistency/viscosity is obtained.

Glycerol may be further added to the composition to arrive at the desired level of viscosity. Glycerol may be added to arrive at a glycerol:OC ratio of about 0.5:1, 0.6:1, 0.7:1, 0.8:1, 0.9:1, 1:1, 1.1:1, 1.2:1, 1.3:1, 1.4:1, 1.5:1, 2:1, 2.5:1, 3:1, 3.5:1, 4:1, 4.5:1, 5:1, or 5.5:1 w/w, including any ratio therebetween.

Glycerol may be added to arrive at a glycerol:OC ratio of between about 0.5:1 and about 6:1 w/w. Glycerol may be added to arrive at a glycerol:OC ratio of between about 0.5:1 and 5:1 w/w; 0.5:1 and 4:1 w/w; 0.5:1 and 3:1 w/w; 0.5:1 and 2:1 w/w; 0.5:1 and 1:1 w/w; or 0.5:1 and 0.9:1 w/w. Glycerol may be added to arrive at a glycerol:OC ratio of between about 0.9:1 and 6:1 w/w; between 1:1 and 6:1 w/w; between 1.5:1 and 6:1 w/w; between 0.9:1 and 5:1 w/w; between 0.9:1 and 4:1 w/w; between 1:1 and 4:1 w/w; between 1.5:1 and 4:1 w/w; or between 1.5:1 and 3:1 w/w.

The term "combining" as used herein relates to adding the OC and the glycerol to each other by any suitable method, including contacting, mixing, blending, stirring, etc.

In some embodiments, the composition has a resistance lower than 20 N as measured in a tensile machine adjusted to monitor a probe having a 1.27 cm diameter at a speed of 30 mm/min from a defined preload of 0.1 N at 8 mm penetration at one or more temperature values selected from the group consisting of: 10°C, 15°C, 20°C, 25°C, 30°C, 35°C, 37°C, and 40°C.

In some embodiments, the composition has a viscosity of at least about 10% higher than that of the glycerol. In some embodiments, the composition has a viscosity lower than about 2.6X10⁹ centipoise (cP) at one or more temperature values selected from the group consisting of 15°C, 20°C, 25°C, 30°C, 35°C, 37°C, and 40°C.

In some embodiments, the composition has a viscosity of at least about 10% higher than that of the glycerol. In some embodiments, the composition has a viscosity lower than about 2.0X10⁴ centipoise (cP) at one or more temperature values selected from the group consisting of 15°C, 20°C, 25°C, 30°C, 35°C, 37°C, and 40°C.

In some embodiments, the composition has a viscosity of at least about 5%, 6%, 7%, 8%, 9%, 10%, 11%, 12%, 13%, 14%, 15%, 16%, 17%, 18%, 19%, or 20% higher than that of glycerol at one or more temperature values selected from the group consisting of 10°C, 15°C, 20°C, 25°C, 30°C, 35°C, 37°C, and 40°C.

In some embodiments, the composition has a viscosity lower than about 5X10⁹, 2.6X10⁹, 1X10⁹, 5X10⁸, 1X10⁸, 5X10⁷, 1X10⁷, 5X10⁶, 1X10⁶ , 5X10⁵, 1X10⁵, 5X10⁴, 2X10⁴, or 1X10⁴ cP at one or more temperature values selected from the group consisting of 10°C, 15°C, 20°C, 25°C, 30°C, 35°C, 37°C, and 40°C.

In some embodiments, the OC comprises oxidized regenerated cellulose (ORC).

In some embodiments, the particle size of the OC is between 10 µm and 2,000µm, optionally between 50 µm and 300 µm.

The method may further comprise adding calcium to the composition.

The method may further comprise adding at least one biologically active agent to the composition.

The method may further comprise adding to the composition one or more excipients selected from the group consisting of sodium chloride, mannitol, albumin, and sodium acetate.

In some embodiments, the ratio of glycerol to OC is between about 0.5:1 and about 6:1 w/w, respectively.

The carboxyl content of the OC is about 9% to about 18% or 12% to about 18%, by weight.

In some embodiments, the composition is flowable at one or more temperature values selected from the group consisting of 10°C, 15°C, 20°C, 25°C, 30°C, 35°C, 37°C, and 40°C.

In some embodiments, the composition is not flowable at one or more temperature values selected from the group consisting of 10°C, 15°C, 20°C, 25°C, 30°C, 35°C, 37°C, and 40°C.

The method may include an additional step of treating the composition to obtain a low bioburden, by methods known in the art such as, heat treatment, radiation treatment, filtration or chemical treatment, e.g., gamma radiation, filtration using a pore size of 0.22 µm or lower, heat sterilization and aseptic field.

The composition may be defined by e.g. its resistance to penetration, which may be measured by various methods, such as the method detailed in **Example 1** below, by using a tensile method.

The composition may also be defined by e.g. its viscosity, which may be measured by various methods, such as the method detailed in **Example 2** below, by using a viscometer. The viscosity value is measured at a certain temperature and may change with temperature.

In **Example 2,** the viscosity was measured for several glycerol:ORC ratios and an extrapolation curve was built. The viscosities of additional glycerol:ORC ratios were calculated using the extrapolation curve.

It is noted that unless indicated otherwise, the viscosity values referred to in the application are as measured, i.e. without subtracting the glycerol background. In the experiments presented hereinbelow, the viscosity of the glycerol background is about 1,504 cP.

In a further aspect, the composition comprises oxidized cellulose (OC) and glycerol, wherein the viscosity of the composition is at least about 10% higher than that of glycerol and lower than about 2.6X10⁹ cP at one or more temperature values selected from the group consisting of: 10°C, 15°C, 20°C, 25°C, 30°C, 35°C, 37°C, and 40°C; the total water content is less than about 8% w/w; and the composition is in the form of a paste at one or more temperature values selected from the group consisting of 10°C, 15°C, 20°C, 25°C, 30°C, 35°C, 37°C, and 40°C.

In some embodiments, the viscosity of the composition is lower than about 2.0X10⁴ cP at one or more temperature values selected from the group consisting of: 10°C, 15°C, 20°C, 25°C, 30°C, 35°C, 37°C, and 40°C.

In some embodiments, the composition has a viscosity of at least about 11%, 12%, 13%, 14%, 15%, 16%, 17%, 18%, 19%, or 20% higher than that of glycerol at one or more temperature values selected from the group consisting of 10°C, 15°C, 20°C, 25°C, 30°C, 35°C, 37°C, and 40°C.

In some embodiments, the composition has a viscosity lower than about 1X10⁹, 5X10⁸, 1X10⁸, 5X10⁷, 1X10⁷, 5X10⁶, 1X10⁶, 5X10⁵, 1X10⁵, 5X10⁴, 2X10⁴, or 1X10⁴ cP at one or more temperature values selected from the group consisting of 10°C, 15°C, 20°C, 25°C, 30°C, 35°C, 37°C, and 40°C.

In some embodiments, a viscosity of about 2.6X10⁹ cP approximately corresponds to a glycerol:OC ratio of about 0.5:1 , and is a highly viscous composition in a paste consistency as defined above, which is a non-flowable paste.

In some embodiments, a viscosity of about 10% higher than that of glycerol approximately corresponds to a viscosity of about 1,650 cP, and to a glycerol:OC ratio a bit higher than 6:1, which is also in a paste consistency as defined above, and is a flowable paste.

Typically, but not exclusively, the cutoff between the flowable paste consistency and the non-flowable paste consistency is at about 3,500 cP. Accordingly, typically, but not exclusively, viscosity of over about 3,500 cP will be considered as non-flowable, while viscosity of under about 3,500 cP would be considered as flowable.

In one embodiment, the cutoff between flowable and non-flowable conditions appear to be between a glycerol:OC w/w ratio of 3.15:1 and 3.78:1. Accordingly, in some embodiments, flowable viscosity values correspond to a glycerol:OC w/w ratio of more than 3.15:1. In some embodiments, non-flowable viscosity values correspond to a glycerol:OC w/w ratio of less than 3.78:1.

In some embodiments, the viscosity of the composition is at least about 1,700 cP. In some embodiments, the viscosity of the composition is at least about 1,800 cP, 1,900 cP, or 2,000 cP.

In some embodiments, the composition has a viscosity of about 3,500 cP or lower at one or more temperature values selected from the group consisting of 10°C, 15°C, 20°C, 25°C, 30°C, 35°C, 37°C, and 40°C. In some embodiments, this viscosity corresponds to a flowable composition.

In some embodiments, the composition has a viscosity of about 3,500 cP or higher at one or more temperature values selected from the group consisting of 10°C, 15°C, 20°C, 25°C, 30°C, 35°C, 37°C, and 40°C. In some embodiments, this viscosity corresponds to a non-flowable composition.

In some embodiments, the carboxyl content of the OC is about 12% to about 18%.

In some embodiments, the composition in which the carboxyl content of the OC is about 12% to about 18%, and the viscosity is about 3,500 cP or lower at one or more temperature values selected from the group consisting of 10°C, 15°C, 20°C, 25°C, 30°C, 35°C, 37°C, and 40°C is used preventing or treating adhesions.

In some embodiments, the composition has a resistance to penetration lower than about 20 N as measured in a tensile machine adjusted to monitor a probe having a 1.27 cm diameter at a speed of 30 mm/min from a defined preload of 0.1 N at 8 mm penetration at about room temperature.

In some embodiments, the composition has a resistance to penetration higher than about 1 N as measured in a tensile machine adjusted to monitor a probe having a 1.27 cm diameter at a speed of 30 mm/min from a defined preload of 0.1 N at 8 mm penetration at about room temperature.

In some embodiments, the ratio of glycerol to OC is at least about 0.5:1 w/w glycerol:OC. In some embodiments, the ratio of glycerol to OC is between about 0.5:1 and about 6:1 w/w glycerol:OC.

In some embodiments, the OC comprises oxidized regenerated cellulose (ORC).

In some embodiments, the composition further comprises at least one biologically active agent. In some embodiments, the at least one biologically active agent is calcium. In some embodiments, the at least one biologically active agent is thrombin.

In some embodiments, the composition further comprises one or more excipients selected from the group consisting of sodium chloride, mannitol, albumin, and sodium acetate.

In a further aspect, the present invention provides a composition comprising oxidized cellulose (OC) and glycerol, wherein the viscosity of the composition is at least 10% higher than that of glycerol and lower than about 2.0X10⁴ cP at one or more temperature values selected from the group consisting of: 10°C, 15°C, 20°C, 25°C, 30°C, 35°C, 37°C, and 40°C; the total water content is less than about 8% w/w; and the composition is in the form of a paste at one or more temperature values selected from the group consisting of 10°C, 15°C, 20°C, 25°C, 30°C, 35°C, 37°C, and 40°C.

In a further aspect, the present invention provides a composition in the form of a paste comprising oxidized cellulose (OC) and glycerol, wherein the ratio of glycerol to OC is at least about 0.5:1 w/w glycerol:OC; the total water content is less than about 8% w/w; and the viscosity of the composition is at least 10% higher than that of glycerol and lower than about 2.6X10⁹ cP at one or more temperature values selected from the group consisting of: 10°C, 15°C, 20°C, 25°C, 30°C, 35°C, 37°C, and 40°C.

In a further aspect, the present invention provides a composition in the form of a paste comprising oxidized cellulose (OC) and glycerol, wherein the ratio of glycerol to OC is at least about 0.5:1 w/w glycerol:OC; the total water content is less than about 8% w/w; and the viscosity of the composition is at least 10% higher than that of glycerol and lower than about 2.0X10⁴ cP at one or more temperature values selected from the group consisting of: 10°C, 15°C, 20°C, 25°C, 30°C, 35°C, 37°C, and 40°C.

Further, in an aspect of the present invention, there is provided a composition according to the claims for use in a method of reducing adhesion formation in tissues and/or organs, e.g., in a patient undergoing surgery. The method comprises applying or contacting the composition at least in the surgical site and/or its proximity.

A further advantage of the compositions of the invention is that they are bioabsorbable, and therefore may be left behind following surgery without causing any side effects. This is in contrast to the currently available compositions used, e.g. to control bleeding in bones, such as bone wax, which is prepared from beeswax which is not bioabsorbable and may impede healing of the tissue.

In an additional aspect, the composition of the present invention may be provided in a kit, wherein the kit comprises: a) a container containing the composition of the invention as described above, b) an applicator for applying the composition to a tissue, and c) optionally instructions for use.

In some embodiments, the contained is part of the applicator.

It is appreciated that the consistency of the composition is such that it can be applied, for example, by spreading or by sticking the composition directly onto the bleeding site. Accordingly, the composition does not need to be further spread on or applied to a solid surface, object, or other solid medium such as a strip or a film in order to be in the appropriate form for applying to the bleeding site. Nevertheless, a suitable applicator, such as, for example, a syringe, may be used in order to apply, spread or stick the composition onto the bleeding site, for the purpose of easy access and handling.

The terms "comprises", "comprising", "includes", "including", "having", and their conjugates mean "including but not limited to". The term "consisting of" means "including and limited to". The term "consisting essentially of" means that the composition, method or structure may include additional ingredients, steps and/or parts, but only if the additional ingredients, steps and/or parts do not materially alter the basic and novel characteristics of the claimed composition, method or structure.

The word "exemplary" is used herein to mean "serving as an example, instance or illustration". Any embodiment described as "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the invention may include a plurality of "optional" features unless such features conflict.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

In those instances where a convention analogous to "at least one of A, B, and C, etc." is used, in general such a construction is intended in the sense one having skill in the art would understand the convention (e.g., "a system having at least one of A, B, and C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Unless otherwise indicated, all numbers such as those expressing, for example, ratios, weight, mole/mole, amounts, viscosity, temperatures, etc., are to be understood as being modified in all instances by the term "about". Accordingly, unless indicated to the contrary, the numerical parameters set forth in this description and attached claims are approximations that may vary by up to plus or minus 10% depending upon the desired properties sought to be obtained by the present invention.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non-limiting fashion.

### Materials and Methods

### Materials

Glycerol was obtained from J.T. Baker (Cat. 2136-01), SURGICEL^{®} (ORC) from Ethicon (Cat. PBM05152017 Mill2), INTERCEED^{®} (ORC) from Ethicon (Cat. IBULK X319 Mill2).

*In vivo porcine punch biopsy model for testing of hemostatic activity:* The animal Procedure used is similar to the protocol previously reported in MacDonald et al. 2017, Medical Devices: Evidence and Research 10: 273-279, with several alterations:
In exemplary procedures, a disposable biopsy punch of 8mm with a stop set to 4 mm was used.

Tamponade was applied for 30 seconds after the test article application and the presence of free-flowing blood was monitored for 2 minutes, and the number of tamponades applied to achieve no free-flowing blood was noted.

Briefly, a ventral midline abdominal incision was performed, and the cranial portion of the midline incision was extended to improve exposure of the liver. The liver was positioned as necessary to maximize testing surface availability. The abdominal organs were kept moist with saline and saline-soaked laparotomy sponges throughout the procedure. The liver parenchymal defects were created on the diaphragmatic surface of accessible areas of the left, right, and quadrate lobes using a disposable 8 mm biopsy punch with a depth stop set to 4 mm. The core portion of the biopsy was grasped and sharply dissected free from the underlying surface causing mild to moderate hemorrhage.

The defect sites were allowed to bleed for several seconds prior to product application to allow for characterization of the resulting hemorrhage. Bleeding at each defect site was classified at a range from 0-5 at the time of wound creation and prior to treatment. Defects classified as 1 or less were excluded and no testing was conducted on them. The trial site was blotted with gauze and then one of the test articles was applied. For the purposes of this study, effective hemostasis was defined as the cessation of free flow bleeding. Pinpoint or petechial bleeding that appeared but did not grow was not considered to be free flow bleeding.

The test article was dispensed onto the defect (bleeding site) either by directly pouring the material onto the defect for the "Dry ORC" composition; by using a syringe for the ORC + glycerol compositions; or by using the Surgiflo applicator for the control articles. An initial tamponade was applied for approximately 30 seconds using digital pressure on a gauze. Following the initial 30 seconds of tamponade, the dressing was removed. If hemostasis was not achieved after 120 seconds, the tamponade was reapplied and maintained for an additional 30 seconds followed by another observation period. This process was repeated until effective hemostasis was achieved, and the number of applied tamponades was recorded.

*In vivo porcine sternotomy model for testing of hemostatic activity in bone bleeding:* After the porcine punch model was concluded, the sternum was exposed, and a median sternotomy was preformed using a bone saw and a sternal retractor to forces apart the bisected sternum.

The resulted persisting bleedings from the bisected sternum were addressed by the manual application of the glycerol + ORC (about 0.5:1 v/w) composition with no tamponade application. The initial bleeding site was monitored for 2 minutes for the presence of free-flowing blood. Two bleeding sites were addressed with the ORC + glycerol composition (about 1:0.5 w/v) and no bleeding was observed following the monitoring step. In addition, it was noted that the removal, by scraping, of the excess ORC + glycerol from the initial bleeding site did not result in additional bleeding.

*In vivo Rat cecal abrasion model for testing for adhesion prevention e activity:* Briefly, animals anesthesia was administered using a single intramuscular injection of a mixture of Ketamine HCl 80 mg/kg (Fort Dodge Pty. Ltd., Australia) and Xylazine HCl 10 mg/kg (VMD, Belgium). (The anesthesia was administered intramuscularly and not intraperitoneally because the operating procedure was performed on the peritoneal cavity).

A 6 cm incision mark was made on the skin overlaying the linea on the ventral midline. The ventral skin was shaved, prepared with iodophor solution and incised. The skin was retracted and undermined slightly to facilitate suturing at the end of the procedure. With the muscle wall exposed, a 5 cm incision in the muscle was made along the linea all through the peritoneal cavity. The right abdominal wall was reflected. A 1x2 cm layer of the peritoneum and part of the muscle was removed. The medial edge of this defect was located 1 cm lateral from the midline incision and parallel to it. The abdominal wall defect was monitored to observe any bleeding. A corresponding defect was made on the cecum, by scraping with a scalpel so that a homogeneous surface of petechial hemorrhages was formed over a lx2cm area. Prior to abrasion, the cecum was elevated and positioned so that upon closure, the cecum would contact the abdominal wall defect to induce local adhesion. The two surfaces were airdried for 10 minutes. For each test article, an amount was spread over the cecum to fully cover the cecum with a thin layer of the test article. The "no treatment" group was left untouched. After the group assignment and application, the cecum and abdominal wall defect were held together for 1 minute prior to closure. Organs were replaced anatomically, and two sutures were placed in each end of the defects to maintain organs proximity. During the treatment, the animals were observed carefully to remove any animal with unexpected response to the anesthetic treatment.

Following the procedure, the animals were given a single dose of Butorphanol Tartrate (Torbugestic) 0.5-2.0 mg/kg body weight to reduce pain. The animals were monitored daily, and in cases where clinical signs or behavior changes were observed, butorphanol 0.5-2.0 mg/kg body weight was administered to the animals subcutaneously every four hours as necessary to control the pain, depending on activity of the animal. fourteen days following surgery, animals were euthanized by CO₂ asphyxiation. The abdomen was opened, and the surgical site inspected. Adhesions were graded by a blinded observer.

The adhesions strength to the various abdominal organs were evaluated according to the following scheme (Poehnert et al., 2015, International journal of medical sciences 12(1):1-6): Grade 0 - no adhesion; Grade 1- filmy adhesions easily removed; Grade 2 - can be removed with blunt dissection; Grade 3 - requires sharp dissection; Grade 4 - highly inseparable with difficulty to differentiate edge of different tissues. In addition to the adhesion strength, adhesion coverage was measured using a ruler. Adhesion intensity was calculated as adhesion strength X adhesion coverage (mm).

### Example 1: Testing the resistance of oxidized regenerated cellulose (ORC) - glycerol combined pastes.

The measurement was conducted by using a LF Plus Tensile Machine (Lloyd Instruments) for monitoring the force exerted during penetration. The Tensile Machine extends a metallic probe into the sample for a preselected distance and registers the resistance, or load, (in Newtons) exhibited by the sample. The probe fitted to the machine was a cylindrical probe with a flat edge having a 1.27 cm diameter. The load cell, which is the sensor monitoring the exerted force, was either 10 N (which can detect a maximum of 10 N force) or 100 N (which can detect a maximum of 100 N force) with accordance to the sample resistance. The Tensile Machine settings were adjusted to monitor an 8 mm penetration starting from a defined preload of 0.1 N. The 0.1 N preload assures that only once 0.1 N resistance was monitored, and the probe is within the sample, the 8 mm penetration registration commenced. The speed of the probe was set to 30 mm/min. Before each test, the sample was centrally located beneath the probe.

Several formulations having various ratios of glycerol to ORC were prepared by adding the respective volumes from **Table 1** of anhydrous glycerol to 4 grams (gr) of dry milled ORC. The formulations were prepared in 3 cm diameter glass vials and were then incubated overnight in a 60-80 °C oven and brought back to room temperature. Following incubation, the formulations were either directly used, or further mixed prior to using.

**Table 1 - glycerol volumes added to 4 gr of dry ORC**

| Paste consistency | Glycerol/ORC ratios (w/w*) | Glycerol volume |
|---|---|---|
| Non-flowable | 3.15:1 | 10 ml |
| Flowable | 3.78:1 | 12 ml |
| Flowable | 4.09:1 | 13 ml |
| Flowable | 4.41:1 | 14 ml |

| | | |
|---|---|---|
| *based on a glycerol density of 1.26 gr/ml | | |

The formulations were tested, with and without manual mixing, for their resistance to a penetrating force as described above. Exact data could not be obtained for the mixed formulation of ratios 4.09:1 and 4.41:1 Glycerol to ORC (w/w) since the low resistance obtained was below the preload setting of the machine.

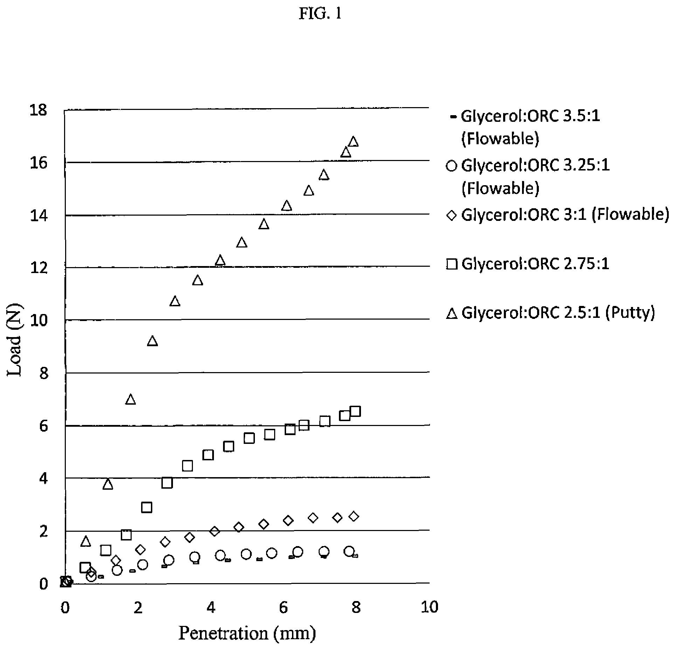

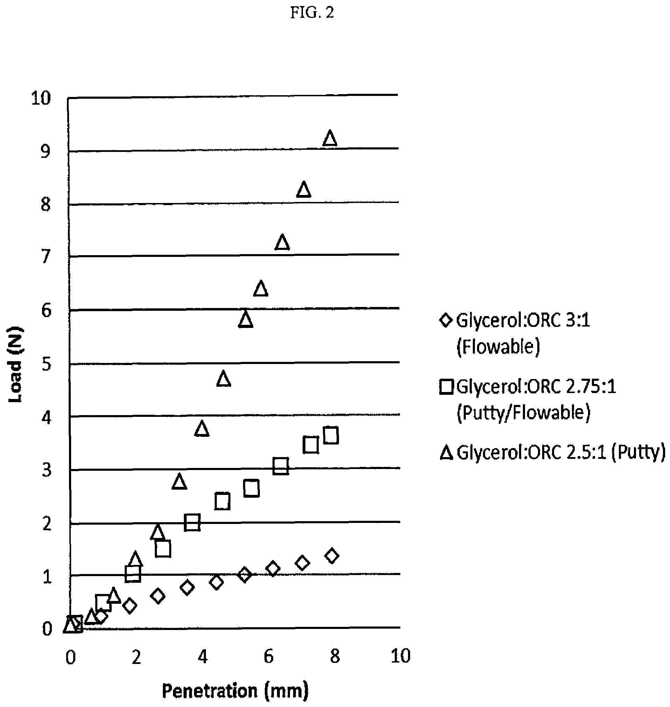

**Figs. 1** **and** **2** demonstrate the penetration resistance of the various paste consistencies. As can be seen from the figures, the putty consistency had a higher resistance to penetration than the more flowable consistencies. The resistance of the non-mixed (Fig. 1) compositions was higher than that of the mixed compositions (Fig. 2). For flowable compositions resistance was as high as just above 3 N for the mixed and just above 6 N for the non-mixed, while for the putty consistence the resistance was just above 9 N for mixed and just above 16 N for non-mixed.

### Example 2: Dynamic viscosity of glycerol:Oxidized Regenerated Cellulose (ORC) mixtures at various ratios

Samples were prepared by mixing milled ORC (prepared by the process described in US9539358) (SURGICEL® or INTERCEED®) with 100% glycerol at various ratios as indicated in **Table 2** (weight/volume: 1:1.5, 1:2, 1:2.5, 1:3, 1:3.5, 1:4) inside a dehumidifier under low humidity (<20%) conditions. After manually mixing, the samples were placed in a 6 ml glass vial (Fiolax clear, lot/#6102981482, Having a 22mm diameter) and homogenized on a roller (Stuart Scientific, Roller Mixer SRT2) for proximally 16-20 Hours at room temperature. A sample having a glycerol:INTERCEED^{®}ORC v/w ratio of 3:1 was gammairradiated at 20 to 40 KGray to reduce bioburden.

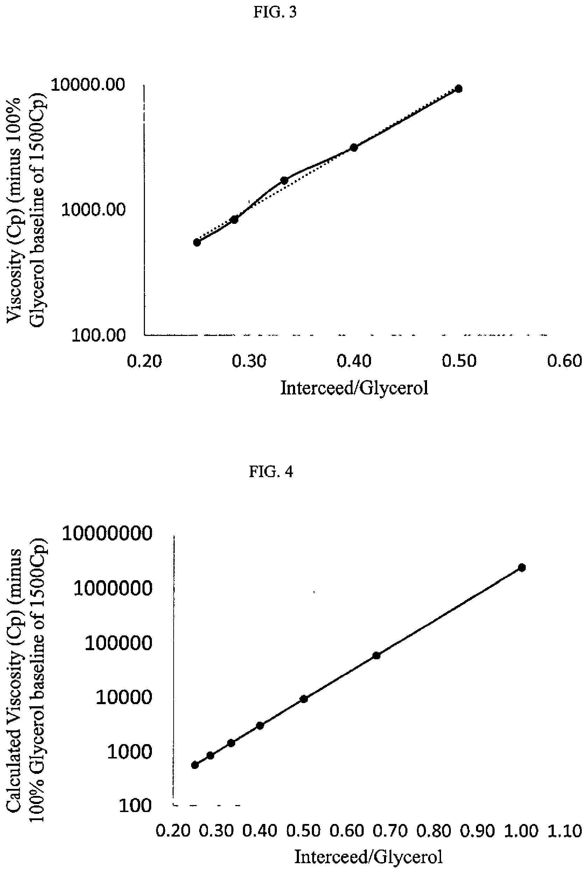

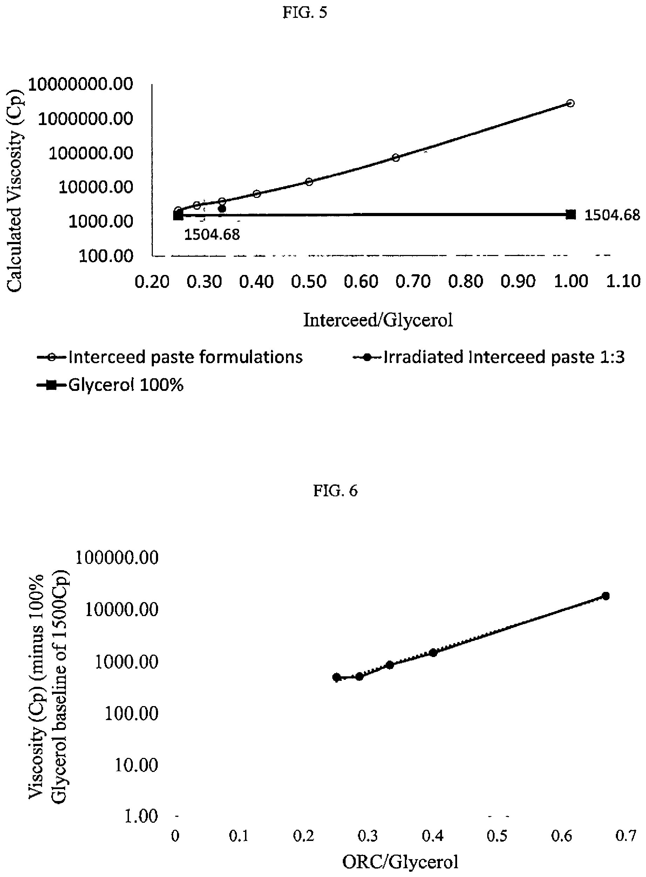

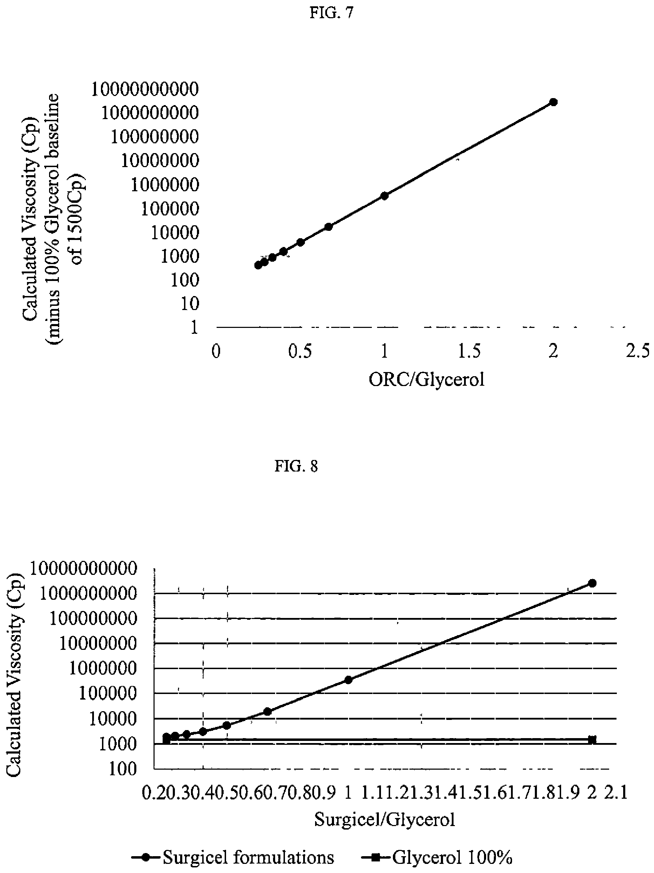

The viscosities of exemplary formulation were measured using a Brookfield viscometer, model LVDV-II+Pro EXTRA with Brookfield HELIPATH Spindle - TF (with diameter of 10.9). All samples were allowed to equilibrate to an approximately 20-22°C for 16-20 hours prior to measurement. Each sample was analyzed using 3 different rotational speeds of 20-200 revolutions per minute (RPM) depending on the sample's viscosity. Each sample had 5 readings and the average was calculated in centipoise (cP). Based on the average readings of the samples, logarithmic charts were created with R squares of over 0.99 and trendlines equations were calculated to extrapolate unmeasured values. For the extrapolation, the results without glycerol background were used. The viscosity results of the various compositions are presented below, and in **Figs. 3-8****.** R-squared (R²) represents the proportion of the variance for a dependent variable that's explained by an independent variable or variables in a regression model.

**Table 2. ORC - Glycerol ratios**

| ORC/glycerol w/v Ratio | Glycerol/ORC w/w ratio | SURGICEL^{®} (gr) | Glycerol (gr) | | INTERCEED^{®} (gr) | Glycerol (gr) |
|---|---|---|---|---|---|---|
| 1 : 4 (0.25) | 5.04:1 | 1 | 5.04 | | 1 | 5.04 |
| 1 : 3.5 (0.29) | 4.41:1 | 2 | 8.82 | | 1 | 4.41 |
| 1 : 3 (0.33) | 3.78:1 | 1 | 3.78 | | 2 | 7.56 |
| 1 : 2.5 (0.4) | 3.15:1 | 2 | 6.3 | | 2 | 6.3 |
| 1 : 2 (0.5) | 2.52:1 | | NA | | 2 | 5.04 |
| 1 : 1.5 (0.67) | 1.89:1 | 2 | 3.78 | | | NA |

**Table 3. INTERCEED^{®}-ORC/glycerol examined viscosity (including background)**

| Glycerol/ORC v/w | Glycerol/ORC w/w | ORC/glycerol w/v | Dynamic Viscosity (cP) |
|---|---|---|---|
| Glycerol 100% | | | 1504.68 |
| 4:1 | 5.04:1 | 0.25 | 2056.12 |
| 3.5:1 | 4.41:1 | 0.29 | 2334.50 |
| 3:1 | 3.78:1 | 0.33 | 3199.94 |
| 2.5:1 | 3.15:1 | 0.40 | 4592.46 |
| 2:1 | 2.52:1 | 0.50 | 10517.29 |

**Table 4. INTERCEED^{®}-ORC/glycerol calculated viscosity (minus background)**

| Glycerol/ORC v/w | Glycerol/ORC w/w | ORC/glycerol w/v | Calculated Dynamic Viscosity (cP) |
|---|---|---|---|
| 4:1 | 5.04:1 | 0.25 | 583 |
| 3.5:1 | 4.41:1 | 0.29 | 867 |
| 3:1 | 3.78:1 | 0.33 | 1473 |
| 2.5:1 | 3.15:1 | 0.40 | 3089 |
| 2:1 | 2.52:1 | 0.50 | 9386 |
| 1.5:1 | 1.89:1 | 0.67 | 59821 |
| 1:1 | 1.26:1 | 1.00 | 2430091 |
| 0.5:1 | 0.63:1 | 2.00 | 162906032094 |

**Table 5. INTERCEED^{®}-ORC/glycerol calculated viscosity (including background)**

| Glycerol/ORC v/w | Glycerol/ORC w/w | ORC/glycerol w/v | Calculated Dynamic Viscosity (cP) |
|---|---|---|---|
| Glycerol 100% | | | 1504.68 |
| 4:1 | 5.04:1 | 0.25 | 2088 |
| 3.5:1 | 4.41:1 | 0.29 | 2372 |
| 3:1 | 3.78:1 | 0.33 | 2977 |
| 2.5:1 | 3.15:1 | 0.40 | 4594 |
| 2:1 | 2.52:1 | 0.50 | 10890 |
| 1.5:1 | 1.89:1 | 0.67 | 61325 |
| 1:1 | 1.26:1 | 1.00 | 2431596 |
| 0.5:1 | 0.63:1 | 2.00 | 162906033599 |
| 3:1 (Irradiated) | 3.78:1 | 0.33 | 2339 |

**Table 6. SURGICEL^{®}-ORC/glycerol examined viscosity (minus background)**

| Glycerol/ ORC v/w | Glycerol/ORC w/w ratio | ORC/glycerol w/v | Dynamic Viscosity (cP) | Paste consistency |
|---|---|---|---|---|
| Glycerol 100% | | | 1504.68 | |
| 4:1 | 5.04:1 | 0.25 | 507.39 | flowable |
| 3.5:1 | 4.41:1 | 0.29 | 520.51 | flowable |
| 3:1 | 3.78:1 | 0.33 | 867.94 | flowable |
| 2.5:1 | 3.15:1 | 0.40 | 1483.75 | non-flowable |
| 1.5:1 | 2.52:1 | 0.67 | 18283.29 | non-flowable |

**Table 7. SURGICEL^{®}-ORC/glycerol calculated viscosity (minus background)**

| Glycerol/ ORC v/w | **Glycerol/ORC w/w ratio** | ORC/glycerol w/v | Calculated Dynamic Viscosity (cP) | Expected Paste consistency |
|---|---|---|---|---|
| 4:1 | 5.04:1 | 0.25 | 424 | flowable |
| 3.5:1 | 4.41:1 | 0.29 | 583 | flowable |
| 3:1 | 3.78:1 | 0.33 | 892 | flowable |
| 2.5:1 | 3.15:1 | 0.40 | 1616 | non-flowable |
| 2:1 | 2.52:1 | 0.50 | 3946 | non-flowable |
| 1.5:1 | 1.89:1 | 0.67 | 17463 | non-flowable |
| 1:1 | 1.26:1 | 1.00 | 342006 | non-flowable |
| 0.5:1 | 0.63:1 | 2.00 | 2569259575 | non-flowable |

**Table 8. SURGICEL^{®}-ORC/glycerol calculated viscosity (including background)**

| Glycerol/ ORC v/w | **Glycerol/ORC w/w ratio** | ORC/glycerol w/v | Calculated Dynamic Viscosity (cP) | Expected paste consistency |
|---|---|---|---|---|
| Glycerol 100% | | | 1504 | |
| 4:1 | 5.04:1 | 0.25 | 1929 | flowable |
| 3.5:1 | 4.41:1 | 0.29 | 2088 | flowable |
| 3:1 | 3.78:1 | 0.33 | 2396 | flowable |
| 2.5:1 | 3.15:1 | 0.40 | 3121 | non-flowable |
| 2:1 | 2.52:1 | 0.50 | 5451 | non-flowable |
| 1.5:1 | 1.89:1 | 0.67 | 18967 | non-flowable |
| 1:1 | 1.26:1 | 1.00 | 343511 | non-flowable |
| 0.5:1 | 0.63:1 | 2.00 | 2569261080 | non-flowable |

### Reference Example 3: SURGICEL^{®}-ORC paste having an ORC/glycerol w/v ratio of 1:1 for stopping bleeding in soft tissues

Compositions were prepared as detailed in **Table 9.**

**Table 9 - preparation of SURGICEL^{®}-ORC compositions**

| Composition | ORC | Glycerol | CaCl₂ | Thrombin |
|---|---|---|---|---|
| Dry ORC | 5 gr | | | |
| ORC/glycerol | 5 gr | 5 ml | | |
| ORC/glycerol + CaCl₂ | 5 gr | 5 ml | 0.5 gr | |
| ORC/glycerol + thrombin | 5 gr | 5 ml | | 800-1200 IU/ml |

The compositions were prepared in the following way:
Dry ORC: 5 gr of SURGICEL^{®}- milled ORC (Ethicon, AC3X112916-1) were weighed into a vial and sealed; ORC + glycerol: 5 gr of milled ORC were weighed into a vial, 5 ml of Glycerol (J.T.Baker, Batch no. 0000136697) were added using a syringe (Yoel Naim, Batch no. 20130815), the vial was sealed and placed on a roller for 24 hours;
ORC + glycerol + CaCl₂: 5 gr of SURGICEL^{®}- milled ORC were weighed into a vial; 0.5 gr of CaCl₂ (Sigma Aldrich, Cat#21097-50G) were added into the same vial, 5 ml of Glycerol were added using a syringe to the vial. Next, the vial was sealed and placed on a roller for 24 hours;
ORC + glycerol + thrombin: 5 gr of SURGICEL^{®}-milled ORC were weighed into a vial, 3 lyophilized thrombin (Omrix, W46T410SD) vials were filled with Glycerol, 2 ml for each vial, placed on a roller, and once the thrombin was dissolved, 5 ml of the Glycerol + thrombin were taken and added to the vial containing the 5 gr of milled ORC. Next, the vial was sealed and placed on a roller for 24 hours.

Two controls articles were used in this test that share similar indications.

Gelatin paste: 8 ml of gelatin paste were prepared according to the Surgiflo^{®} (Ethicon, Lot: 248177) manufacturer's instructions for use wherein the paste was mixed with 2 ml of water.

Gelatin paste + thrombin: The 8 ml of gelatin paste was prepared according to the Surgiflo^{®} (Ethicon, Lot: 248177) manufacturer's instructions for use wherein 800-1200 International Units (IU) of thrombin was reconstituted with 2 ml of water, and mixed with the gelatin paste.

The compositions including SURGICEL^{®} ORC and Glycerol were loaded into several 1 ml syringes for easy application and were used in the porcine punch biopsy model.

The hemostatic activity results for the various compositions are presented in Fig. 9.

As can be seen from Fig. 9, all ORC/glycerol compositions tested showed effective hemostatic activity that was better than dry **SURGICEL^{®}-ORC** (milled ORC) or than gelatin paste without thrombin.

### Reference Example 4: SURGICEL^{®}-ORC non-flowable paste having a glycerol/ORC ratio of about 0.5:1 v/w for stopping bleeding in bone tissue

Compositions were prepared as detailed in Table 10.

**Table 10 - preparation of ORC compositions**

| Composition | ORC | Glycerol |
|---|---|---|
| Dry ORC | 5g | |
| ORC/glycerol | 5g | 2.5 ml |

The compositions were created in the following way:
Dry ORC: 5 gr of SURGICEL^{®}- milled ORC having a carboxyl content of 18-21 % (Ethicon, AC3X112916-1) were weighed into a vial and sealed.
ORC + glycerol: 5 gr of SURGICEL^{®}-milled ORC were weighed into a vial, 2.5 ml of Glycerol (J.T.Baker, Batch no. 0000136697) were added using a syringe (Yoel Naim, Batch no. 20130815) to the vial, which was sealed and placed on a roller for 24 hours.
Control articles were prepared as in **Example 3.**

The ORC/glycerol composition showed a hemostatic effect that was better than that of gelatin paste, or that of dry ORC in the punch biopsy model described in the previous example **(see** **Fig. 9****).** The ORC/glycerol composition was also able to achieve full hemostasis in bone tissue, as was tested in the sternotomy model (data not shown).

### Example 5: Adhesion prevention efficacy in a rat cecal abrasion model

Adhesions are a substantial medical problem which can cause chronic pain, infertility and are related to intestinal obstruction that could lead to mortality. Many of these adhesions results from surgical trauma to the peritoneum. To examine the ability of the compositions of the invention to reduce adhesions, they were tested in an animal model. The chosen modal is a Rat model in which includes cecal abrasion and an abdominal sidewall defect. The model is generally described in Poehnert et al., 2015, International Journal of Medical Sciences 12(1):1-6, with a few minor alterations.

The test articles were prepared as follows:
Group No treatment: no treatment.
Group Interceed: commercially available INTERCEED^{®} sheets (Ethicon) was cut to size to be applied to cover the cecum.
Group INTERCEED^{®} powder is in the form of aggregates and were generated by the process described in US9539358 examples with the difference that the base material used was INTERCEED^{®} sheets (Ethicon) instead of SURGICEL^{®} sheets (Ethicon). Interceed powder in the form of aggregates is also named herein: milled /ground INTERCEED in aggregated form, milled/ground INTERCEED^{®} which was compressed into small granules, compacted intercede powder, interceed compacted in the form of aggregate.

Groups 3:1 and 2:1 glycerol:Interceed v/w: the formulation was prepared as described in **Table 2** above, and was further subjected to 20-45 kilogray of gamma radiation (By Sorvan radiation ltd to provide sterility.

Group Glycerol: pure glycerol (J.T. Baker, 2136-01) was used.

The test articles were applied (6 repetitions) to the rat cecal abrasion model as explained in the methods section above. As can be seen from **Fig. 10****,** the 3:1 glycerol:interceed group had the lowest intensity of adhesion prevention , and the 2:1 glycerol:interceed group had an intensity comparable to the Interceed milled ORC group.

It is noteworthy that gamma radiated interceed powder ORC exhibited adhesion prevention potency of about 135% as compared to ORC fabric.

## Claims

1. A composition comprising oxidized cellulose (OC) for use as an adhesion prevention material by topical application of the composition at a surgical site, wherein the OC is in the form of a powder and has a carboxyl content ranging from about 9% to about 18% by weight, and wherein the composition is suitable for preparing a spreadable paste or powder.

2. The composition for use according to claim 1, wherein the carboxyl content is about 12% to about 18% by weight.

3. The composition for use according to claim 1 or claim 2, wherein the OC comprises oxidized regenerated cellulose (ORC).

4. The composition for use according to claim 3, wherein the ORC has a carboxyl content ranging from about 9.5% to about 10.5%.

5. The composition for use according to any preceding claim, wherein the OC in the form of aggregates.

6. The composition for use according to any preceding claim, further comprising at least one biologically active agent.

7. The composition for use according to claim 6, wherein said at least one biologically active agent is calcium.

8. The composition for use according to any preceding claim, further comprising one or more excipients selected from the group consisting of sodium chloride, mannitol, albumin, and sodium acetate.

## Patentansprüche

1. Zusammensetzung, umfassend oxidierte Cellulose (OC) zur Verwendung als ein adhäsionsverhinderndes Material durch topische Anwendung der Zusammensetzung an einer chirurgischen Stelle, wobei die OC in der Form eines Pulvers vorliegt und einen Carboxylgehalt, der von etwa 9 Gew.-% bis etwa 18 Gew.-% reicht, aufweist, und wobei die Zusammensetzung zum Herstellen einer streichbaren Paste oder eines Pulvers geeignet ist.

2. Zusammensetzung zur Verwendung nach Anspruch 1, wobei der Carboxylgehalt etwa 12 Gew.-% bis etwa 18 Gew.-% beträgt.

3. Zusammensetzung zur Verwendung nach Anspruch 1 oder 2, wobei die OC oxidierte regenerierte Cellulose (ORC) umfasst.

4. Zusammensetzung zur Verwendung nach Anspruch 3, wobei die ORC einen Carboxylgehalt, der von etwa 9,5 % bis etwa 10,5 % reicht, aufweist.

5. Zusammensetzung zur Verwendung nach einem der vorstehenden Ansprüche, wobei die OC in der Form von Aggregaten vorliegt.

6. Zusammensetzung zur Verwendung nach einem der vorstehenden Ansprüche, ferner umfassend mindestens ein biologisch aktives Mittel.

7. Zusammensetzung zur Verwendung nach Anspruch 6, wobei das mindestens eine biologisch aktive Mittel Calcium ist.

8. Zusammensetzung zur Verwendung nach einem der vorstehenden Ansprüche, ferner umfassend einen oder mehrere Hilfsstoffe, die aus der Gruppe ausgewählt sind, bestehend aus Natriumchlorid, Mannit, Albumin und Natriumacetat.

## Revendications

1. Composition comprenant de la cellulose oxydée (OC) pour une utilisation en tant que matériau de prévention des adhérences par application topique de la composition au niveau d'un site chirurgical, dans laquelle l'OC est sous la forme d'une poudre et a une teneur en carboxyle allant d'environ 9 % à environ 18 % en poids, et dans laquelle la composition est adaptée à la préparation d'une pâte pouvant être étalée ou d'une poudre.

2. Composition pour une utilisation selon la revendication 1, dans laquelle la teneur en carboxyle est d'environ 12 % à environ 18 % en poids.

3. Composition pour une utilisation selon la revendication 1 ou la revendication 2, dans laquelle l'OC comprend de la cellulose régénérée oxydée (ORC).

4. Composition pour une utilisation selon la revendication 3, dans laquelle l'ORC a une teneur en carboxyle allant d'environ 9,5 % à environ 10,5 %.

5. Composition pour une utilisation selon l'une quelconque revendication précédente, dans laquelle l'OC est sous la forme d'agrégats.

6. Composition pour utilisation selon l'une quelconque revendication précédente, comprenant en outre au moins un agent biologiquement actif.

7. Composition pour une utilisation selon la revendication 6, dans laquelle ledit au moins un agent biologiquement actif est le calcium.

8. Composition pour une utilisation selon l'une quelconque revendication précédente, comprenant en outre un ou plusieurs excipients choisis dans le groupe constitué de chlorure de sodium, mannitol, albumine, et acétate de sodium.
